# EUROPEAN PATENT SPECIFICATION

(11) **EP 2 981 527 B1**
(45) Date of publication and mention of the grant of the patent: **31.10.2018**
(21) Application number: 14717070.8
(22) Date of filing: 07.04.2014
(51) Int. Cl.: C07D 277/64, C12Q 1/66

(54) **DERIVATIVES OF LUCIFERIN.**
LUCIFERIN DERIVATE.
DÉRIVÉS DE LUCIFERIN.

(30) Priority: 05.04.2013 GB 201306248
(43) Date of publication of application: 10.02.2016
(73) Proprietor: University College Cardiff Consultants Limited, South Glamorgan CF24 0DE (GB)
(72) Inventor: JATHOUL, Amit P., London W1T 4TP (GB); GROUNDS, Helen, London SE21 8HP (GB); ANDERSON, James C., Nottingham NG13 0FE (GB); PULE, Martin A., London W1T 4TP (GB)
(74) Representative: Elsworth, Jon David
(86) International application number: PCT/GB2014/051080
(87) International publication number: WO 2014/162157

(56) References cited:
- WO-A1-2013/027770
- S. IWANO ET. AL.: "Development of simple firefly luciferin analogs emitting blue, green, red and near-infrared biological window light.", TETRAHEDRON, vol. 69, 18 March 2013 (2013-03-18), pages 3847-3856, XP002724138, cited in the application

## Description

The present invention relates to compounds and their uses. In particular, though not exclusively, it concerns luminogenic compounds and their application in *in vivo* imaging techniques.

Catalysis of a substrate (e.g. luciferin) by a bioluminescence (BLI) enzyme (e.g. luciferase) results in the release of light. Thus, small animal bioluminescence imaging utilises emission of light by the transgenic expression, particularly of firefly luciferase (Flue), in selected tissue(s). This rapid, cheap and highly sensitive modality has revolutionised the use of small animal models, allowing facile longitudinal and quantitative tracking of labelled cells noninvasively. Furthermore, functionalised substrates and enzymes allow the imaging of biological events within the small animal.

However, it has proven difficult to extend this modality to allow true tomographic and multi-parametric imaging, primarily because luciferases emit light (yellow-green colour) that is largely absorbed by haemoglobin. This causes scatter and differential attenuation depending on tissue density and light source depth. Shifting the emission of luciferase into the near infra-red (nIR) would therefore overcome these barriers, since even complex mammalian tissues are quite transparent at these wavelengths (in the 'optical window' between ca. 620-800 nm).

Despite much interest, no genetically encoded nIR BLI label currently exists. Furthermore, because BLI is currently restricted to relatively small animals, nIR emission with a respectable quantum yield would allow BLI in larger animals than is currently possible.

Firefly luciferase (Flue) has been heavily engineered, increasing its expression, stability and shifting its emission with natural firefly luciferin from green-yellow to red. However, it is apparent that current systems are near the limit of red-shifting (ca. 620nm) achievable by mutagenesis of *Photinus pyralis* Flue.

Firefly luciferin (often denoted LH₂) is known to produce at least two colours of bioluminescence with different mutants of firefly luciferase, the natural yellow-green colour (peak wavelength - λmax 557nm) and a red colour (λmax 620nm). Red mutants are useful for *in vivo* imaging since the attenuation of red light is less intense and variable. In addition, due to the dependence of photon scatter on wavelength (scatter is proportional to 1/wavelength⁴), red emission is expected to allow higher resolution and deeper tissue bioluminescence imaging.

Due to the differential attenuation of the two colours and variable attenuation of the yellow green emission (depending on depth, haemoglobin concentration and tissue density) multiparametric bioluminescence *in vivo* imaging has thus far been impossible. For this, the information space above 600nm (the optical window above haemoglobin absorbance) must be utilised. Therefore, emission should ideally be shifted ca. 100 nm compared to current systems, into the far red or near infrared regions. It would also be beneficial for the maximum emission to be at different wavelengths with different mutants, and to have a narrow band of emission to allow for multiparametric imaging.

The chemical mechanism for the different colours observed with different mutant Flue and native LH₂ is unknown and many studies with mutants and substrate analogues have tried to determine it without success (Navizet et al., J Am Chem Soc, 2010, 132, 706-12). One hypothesis is that the emitting product of the Flue reaction, oxyluciferin (OL) can exist in two tautomeric forms, i.e. red emitting 4-keto and yellow-green emitting 4-enolate (White et al., Bioorg Chem, 1971, 1, 92-122.); however, this theory is less probable since preadenylated 5-substituted dimethyl analogues (which cannot form an enolate) can produce green and red colours with wild-type and red mutant Flues, respectively (Branchini et al., J Am Chem Soc Comm, 2002, 124, 2112-3).

A number of other theories exist, including one solution that the entire molecule can exist in two different resonance forms dictated by electrostatic and/or H-bond interactions in the active site (Branchini et al., Biochemistry, 2004, 43, 7255-62). Although strictly unproven, this theory is supported by quantum mechanical calculations (Nakatani et al., J Am Chem Soc, 2007, 129, 8756-65). Furthermore, the postulated mechanism that leads to emission with LH₂ (chemically induced electron-exchange luminescence - CIEEL) supports the notion of electron movement from 4- to 6'- groups (Koo et al., PNAS, 1978, 75, 30-3). However, it seems probable that the limit of red-shift achievable by enzyme engineering alone has been reached (Wood, Biolum Chemilum, 1990, 5, 107-14).

The theoretical chemical mechanisms for resonance of luciferin are as follows:

### 1. Keto-enolate tautomeric forms of oxyluciferin (OL)

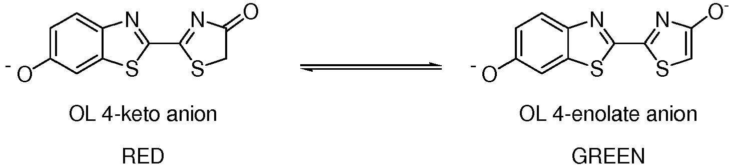

### 2. Charge-based resonance delocalisation of oxyluciferin (OL)

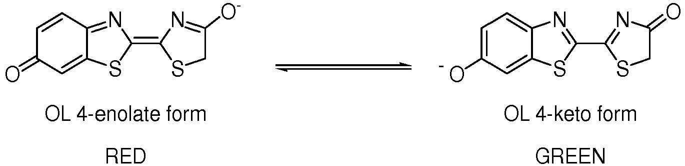

As the limit of red-shift available by enzyme engineering is thought to have been reached, modification of the chemical structure of luciferin (LH₂) is an alternative option. A number of analogues of firefly LH₂ have been previously synthesised to test theories of emission, to attempt to improve light yield or to shift emission colours. However, all have shown reduced light yields compared to native LH₂, and none has shifted emission sufficiently into the far red or near infrared regions. Thus, there is still a need for compounds which do not reduce the light yield too significantly, and which shift the light emission into a region that can be potentially useful outside of the window of haemoglobin absorbance.

Aminoluciferins, including those with the structures illustrated below, have been reported to have a higher affinity for Flue than LH₂ (flash kinetics), lower light emission than LH₂ and lower cell permeability in *in vitro* studies (Maki, J Electrochem Soc Japan, 2006, 9; Iwano et al., Tetrahedron 2013, http://dx.doi.org/10.1016/j.tet.2013.03.050; Ugarova et al., Luminescence 2002, 17, 321-30; Japanese patent publication JP2009184932).

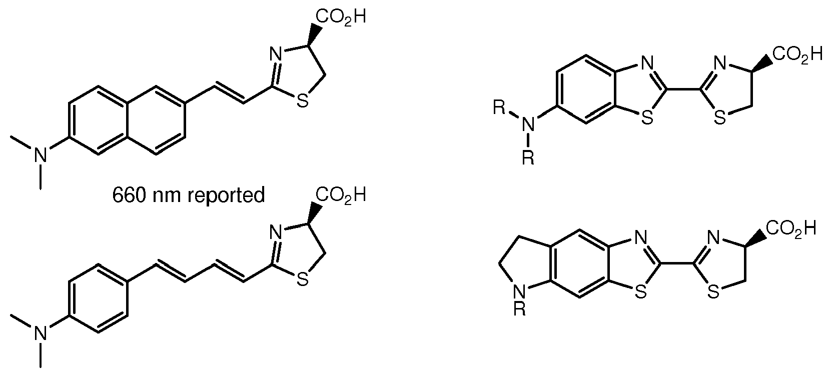

However, no data on the effect of pH on the emission wavelength maxima, or the use of mutated Flue to change the emitted wavelength have been reported. In addition, no *in vivo* studies have been reported.

WO 2013/027770, JP 2010/189359 and CN 102617536 each disclose alternative heterocyclic compounds which do not provide a significant red-shift in emission or allow multiparametric imaging.

Therefore, it is an object of the present invention to provide novel substrate analogues of luciferin that result in red-shifting of emission, particularly to the nIR range. Furthermore, it is desirable for such compounds to possess similar biocompatibility (e.g. good biodistribution and low toxicity) to natural luciferin, and to exhibit different emission colours (have distinct emitting forms) with different Flue mutants.

According to the invention, there is provided a compound according to the formula (II): 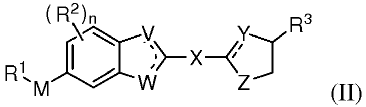 wherein
R¹ is hydrogen or an optionally substituted alkyl group;
each R², when present, is a group independently selected from optionally substituted hydroxyl, optionally substituted amino, optionally substituted thiol, optionally substituted alkyl, and optionally substituted aryl;
R³ is a carboxyl group or an optionally substituted C₁₋₆ alkyl carbonyloxy group;
M is a group selected from O, S, and NR⁴, wherein R⁴ is a group selected from hydrogen, optionally substituted alkyl, optionally substituted alkenyl, optionally substituted aryl, optionally substituted heteroaryl, and optionally substituted heterocyclyl;
X is a group selected from optionally substituted alkenylene, optionally substituted alkynylene, optionally substituted arylene, and combinations thereof, or optionally substituted alkylene carbonyl;
n is 0, 1, 2, or 3;
V and Y are each independently selected from CR⁵ and N;
W and Z are each independently selected from NR⁷, S, and O;
R⁵ is a group selected from hydrogen, optionally substituted hydroxyl, optionally substituted alkyl, and optionally substituted amino; and
R⁷ is a group selected from hydrogen, optionally substituted alkyl, and optionally substituted aryl.

The compounds of formula (II) have been found to represent bioluminescent substrate analogues of luciferin which exhibit a red-shift in emission to the nIR range. Thus, they are the first examples of nIR BLI labels having true bioluminescence peaking in the nIR (i.e. not the product of resonance energy transfer or filtering). Structural features, such as the unsaturated linker group X, further allow different emission colours (have distinct emitting forms) with different mutant enzymes and/or under varying pH conditions.

Such analogues have the potential to enable a new era in multispectral BLI, facilitating higher resolution multi-parametric imaging and enhanced depth penetration, and including the imaging of larger animals, such as dogs, cats, primates and human subjects.

The compounds of formula (II) have been found to be active *in vivo,* for example when injected into mice containing systemic Raji cell-derived lymphomas. The light generated could be imaged for analytical purposes. The compounds therefore provide the potential to increase depth penetration and resolution of BLI by reducing haemoglobin (Hb) attenuation and scatter (a higher percentage of photons are not absorbed by Hb and also travel for longer in a straight path), as well as high sensitivity multispectral BLI when used alone, or in combination with natural luciferin or further analogues.

The term 'C_{x-y} alkyl' as used herein refers to a linear or branched saturated hydrocarbon group containing from x to y carbon atoms. For example, C₁₋₆ alkyl refers to a linear or branched saturated hydrocarbon group containing from 1 to 6 carbon atoms. Examples of C₁₋₆ alkyl groups include methyl, ethyl, n-propyl, isopropyl, n-butyl, isobutyl, sec-butyl, tert-butyl, n-pentyl, isopentyl, neopentyl, hexyl, and isohexyl.

The term 'C_{x-y} alkylene' as used herein refers to a divalent hydrocarbon group obtained by removing one hydrogen atom from 'C_{x-y} alkyl' above. Examples of C₁₋₆ alkylene groups include methylene, ethylene, propylene, butylene, pentylene, and hexylene.

The term 'C_{x-y} alkenyl' as used herein refers to a linear or branched hydrocarbon group containing one or more carbon-carbon double bonds and having from x to y carbon atoms. Examples of C₂₋₆ alkenyl groups include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, and 5-hexenyl.

The term 'C_{x-y} alkenylene' as used herein refers to a divalent hydrocarbon group obtained by removing one hydrogen atom from 'C_{x-y} alkenyl' above. Examples of C₂₋₆ alkenylene groups include ethenylene, propenylene, butenylene, 1,3-butadienylene, pentenylene, hexenylene, and 1,3,5-hexatrienylene.

The term 'C_{x-y} alkynyl' as used herein refers to a divalent hydrocarbon group containing one or more carbon-carbon triple bonds and having from x to y carbon atoms. Examples of C₂₋₆ alkynyl groups include ethynyl, propynyl, butynyl and pentynyl.

The term 'C_{x-y} alkynylene' as used herein refers to a divalent hydrocarbon group obtained by removing one hydrogen atom from 'C_{x-y} alkynyl' above. Examples of C₂₋₆ alkynylene groups include ethynylene, propynylene, butynylene, 1,3-butadiynylene, pentynylene, hexynylene, and 1,3,5-hexatriynylene.

The term 'C_{x-y} alkoxy' as used herein refers to an -O-C_{x-y} alkyl group wherein C_{x-y} alkyl is as defined herein. Examples of C₁₋₆ alkoxy groups include methoxy, ethoxy, propoxy, *iso-*propoxy, butoxy, *tert*-butoxy, pentoxy and hexoxy.

The term 'x- to y-membered hydrocarbon ring' as used herein refers to a cycloalkyl, cycloalkenyl, or aryl ring as defined below in relation to formula (II).

The term 'x- to y-membered cycloalkyl' as used herein refers to a saturated monocyclic hydrocarbon ring of x to y carbon atoms. For example, 3- to 6-membered cycloalkyl refers to a saturated monocyclic hydrocarbon ring of 3 to 6 carbon atoms. Examples of 3- to 6-membered cycloalkyl groups include cyclopropyl, cyclobutyl, cyclopentyl, and cyclohexyl.

The term 'x- to y-membered cycloalkenyl' as used herein refers to a monocyclic hydrocarbon ring containing one or more carbon-carbon double bonds of x to y carbon atoms. For example, C₃₋₆ cycloalkenyl refers to an unsaturated monocyclic hydrocarbon ring of 3- to 6-carbon atoms. Examples of C₃₋₆ cycloalkenyl groups include 2-cyclopenten-1-yl, 3-cyclopenten-1-yl, 2-cyclohexen-1-yl, and 3-cyclohexen-1-yl.

The term 'aryl' as used herein refers to a 5- to 6-membered monocyclic hydrocarbon ring containing x to y carbon atoms, wherein the ring is aromatic. An example of such an aryl group is phenyl. Alternatively, the term 'C_{x-y} aryl' as used herein refers to a monocyclic or bicyclic ring containing from x to y carbon atoms, wherein at least one ring is aromatic. Examples of C₆₋₁₄ aryl groups include phenyl, naphthyl, tetrahydronaphthalenyl, anthryl, phenanthryl, acenaphthylenyl, biphenylyl, anthracenyl, phenanthrenyl, and phenalenyl.

The term 'arylene' as used herein refers to a divalent hydrocarbon group obtained by removing one hydrogen atom from 'C_{x-y} aryl' above. An example of such an arylene group is phenylene. Alternatively, the term 'C_{x-y} arylene' as used herein refers to a monocyclic or bicyclic ring containing from x to y carbon atoms, wherein at least one ring is aromatic. Examples of C₆₋₁₄ arylene groups include phenylene, naphthylene, tetrahydronaphthalenylene, anthrylene, phenanthrylene, acenaphthylenylene, biphenylylene, anthracenylene, phenanthrenylene, and phenalenylene.

The term 'heteroaryl' as used herein refers to a 5- or 6-membered monocyclic aromatic ring in which the monocyclic ring contains 1 to 4 heteroatoms selected from oxygen, nitrogen, and sulphur. Examples of such monocyclic aromatic rings include thienyl, furyl, furazanyl, pyrrolyl, triazolyl, tetrazolyl, imidazolyl, oxazolyl, thiazolyl, oxadiazolyl, isothiazolyl, isoxazolyl, thiadiazolyl, pyranyl, pyrazolyl, pyrimidyl, pyridazinyl, pyrazinyl, pyridyl, triazinyl, and tetrazinyl.

The term 'heterocyclyl' refers to a 5- or 6-membered monocyclic ring which may be saturated or partially unsaturated, in which the monocyclic ring contains 1 to 4 heteroatoms selected from oxygen, nitrogen, and sulphur. Examples of such monocyclic rings include aziridinyl, oxiranyl, pyrrolidinyl, azetidinyl, pyrazolidinyl, oxazolidinyl, piperidinyl, piperazinyl, morpholinyl, thiomorpholinyl, thiazolidinyl, hydantoinyl, valerolactamyl, oxiranyl, oxetanyl, dioxolanyl, dioxanyl, oxathiolanyl, oxathianyl, dithianyl, dihydrofuranyl, tetrahydrofuranyl, dihydropyranyl, tetrahydropyranyl, tetrahydropyridinyl, tetrahydropyrimidinyl, tetrahydrothiophenyl, tetrahydrothiopyranyl, diazepanyl and azepanyl.

The term 'amino' as used herein refers to an organonitrogen compound with the connectivity -N(R')(R"), where R' and R" are each independently a hydrogen or an optional substituent as defined below in relation to formula (II).

The term 'C_{x-y} alkyl carbonyloxy' as used herein refers to an alkyl group wherein C_{x-y} alkyl is as defined herein and at least one methylene group (i.e. -CH₂-) is replaced with an ester group (e.g. -CO₂-). Examples of C₁₋₆ alkyl carbonyloxy groups include ethanoate, propanoate, butanoate, pentanoate, and hexanoate. The term 'carbonyloxy' as used herein refers to a single carbonyloxy group of the formula: -CO₂-.

The term 'C_{x-y} alkyl carbamoyl' as used herein refers to an alkyl group wherein C_{x-y} alkyl is as defined herein and at least one methylene group (i.e. -CH₂-) is replaced with an amide group (e.g. -C(O)NR-, where R is a hydrogen atom, a 5- or 6-membered heterocyclyl group, a 5- or 6-membered heteroaryl group, a 3- to 6-membered cycloalkyl group, a C₁₋₆ alkyl group, or a C₆₋₁₄ aryl group, preferably a hydrogen atom). Examples of C₁₋₆ alkyl carbamoyl groups include ethyl carbamoyl, propyl carbamoyl, butyl carbamoyl, pentyl carbamoyl, and hexyl carbamoyl. The term 'carbamoyl' as used herein refers to a single carbamoyl group of the formula: -C(O)NR-, where R is as defined above.

The term 'C_{x-y} alkoxy carbonyl' as used herein refers to an alkyl group wherein C_{x-y} alkyl is as defined herein and at least one methylene group (i.e. -CH₂-) is replaced with an ester group (e.g. -OC(O)-). Examples of C₁₋₆ alkyl carbonyl groups include ethyl oxycarbonyl, propyl oxycarbonyl, butyl oxycarbonyl, pentyl oxycarbonyl, and hexyl oxycarbonyl. The term 'oxycarbonyl' as used herein refers to a single oxycarbonyl group of the formula: -OC(O)-.

The term 'C_{x-y} alkyl carbonyl' as used herein refers to an alkyl group wherein C_{x-y} alkyl is as defined herein and at least one methylene group (i.e. -CH₂-) is replaced with a carbonyl group (i.e. >C=O). Examples of C₁₋₆ alkyl carbonyl groups include ethanoyl, propanoyl, butanoyl, pentanoyl, and hexanoyl. The term 'carbonyl' as used herein refers to a single carbonyl group of the formula: >C=O.

The term 'hydroxyl' as used herein refers to a group of the formula -OH. If such a group is substituted, the resulting group is an ether of the formula -O-.

The term 'thiol' as used herein refers to a group of the formula -SH. If such a group is substituted, the resulting group is a thioether of the formula -S-.

Each symbol in formula (II) is described in detail in the following.

R¹ is hydrogen or an optionally substituted alkyl group. In a preferable embodiment, R¹ is hydrogen or an optionally substituted C₁₋₃ alkyl group. In particular, for reasons of improved resonance, and thus stability, R¹ is preferably hydrogen.

Each R², when present, is a group independently selected from optionally substituted hydroxyl, optionally substituted amino, optionally substituted thiol, optionally substituted alkyl, and optionally substituted aryl. In a preferable embodiment, R², when present, is a group independently selected from optionally substituted hydroxyl, optionally substituted amino, and optionally substituted alkyl. In particular, for reasons of improved resonance, R² is preferably a group independently selected from hydroxyl and optionally substituted amino.

R³ is optionally substituted C₁₋₆ alkyl carbonyloxy or carboxyl. In particular, when R³ is a C₁₋₃ alkyl carbonyloxy group, a more stable compound is obtained, e.g. racemisation at the α-position to the carbonyl is reduced, and without any significant adverse effects in terms of bioluminescence. Indeed, common esterases cleave such groups *in vivo* to produce functional luciferin analogues having carboxyl functionality. Therefore, although R³ is preferably hydrogen for reasons of improved resonance stability, R³ is preferably a C₁₋₃ alkyl carbonyloxy group for stability and handling *ex vivo* with the ester group being cleaved *in vivo.*

M is a group selected from O, S, and NR⁴, wherein R⁴ is a group selected from hydrogen, optionally substituted alkyl, optionally substituted alkenyl, optionally substituted aryl, optionally substituted heteroaryl, and optionally substituted heterocyclyl. In a preferable embodiment, M is selected from O and S. In particular, M is preferably O. Furthermore, it has been found that compounds wherein M is O and R¹ is hydrogen are particularly stable.

X is a group selected from optionally substituted alkenylene, optionally substituted alkynylene, and optionally substituted arylene groups, and combinations thereof, or an optionally substituted alkylene carbonyl group. In another preferred embodiment, X is a group selected from optionally substituted alkenylene, optionally substituted alkynylene, optionally substituted aralkylene, and optionally substituted alkylene carbonyl. In particular, a C₂₋₆ alkylene carbonyl group, or a group selected from C₂₋₆ alkenylene, C₂₋₆ alkynylene, C₆₋₁₄ arylene, and combinations thereof, is preferred. Most preferably, X is an optionally substituted alkenylene group. Preferably, X provides conjugation between the two ring systems, such that resonance stabilisation is provided. Furthermore, additional conjugation provided by X, i.e. by multiple unsaturated bonds such as -C₂H₂-C₂H₂-, has been found to further red-shift the emission wavelength into the nIR.

The number of possible substituents, R², on the phenyl ring of the compound of formula (II) is denoted n, where n is 0, 1, 2, or 3. In a preferable embodiment, n is 0, 1, or 2, even more preferably n is 0 or 1.

R⁵ is a group selected from hydrogen, optionally substituted hydroxyl, optionally substituted alkyl, and optionally substituted amino. R⁷ is a group selected from hydrogen, optionally substituted alkyl, and optionally substituted aryl.

V and Y are each independently selected from CR⁵ and N. W and Z are each independently selected from NR⁷, S, and O.

Preferably, there is provided a compound of formula (II): 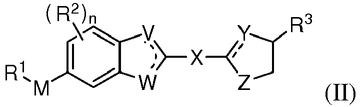 wherein
R¹ is hydrogen or optionally substituted alkyl (preferably hydrogen);
R², when present, is a group independently selected from optionally substituted hydroxyl, optionally substituted amino, and optionally substituted alkyl;
R³ is optionally substituted C₁₋₆ alkyl carbonyloxy or carboxyl;
M is selected from O, S, and NR⁴, wherein R⁴ is a group selected from hydrogen, optionally substituted alkyl, and optionally substituted aryl (preferably O);
V and Y are each independently selected from CR⁵ and N;
W and Z are each independently selected from NR⁷, S, and O,
wherein R⁵ is a group selected from hydrogen, optionally substituted hydroxyl, optionally substituted alkyl, and optionally substituted amino, and
R⁷ is a group selected from hydrogen, optionally substituted alkyl, and optionally substituted aryl; and
n is 0 or 1.

Such compounds have been shown to possess an emission wavelength in the nIR, and therefore show improved spectral properties through haemoglobin, i.e. there is less interference and/or scatter when imaging is conducted *in vivo.* In addition, the compounds exhibit an excellent quantum yield, at least comparable to those known in the art, yet also provide an additional red-shift with Flue colour mutants.

In another preferred embodiment of the invention, there is provided a compound having the formula (III): 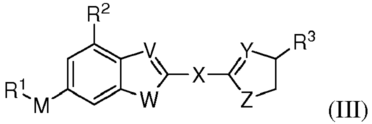 wherein
V and Y are each independently selected from CR⁵ and N;
W and Z are each independently selected from NR⁷, S, and O; and
the remaining groups are as defined above in relation to formula (II).

In this embodiment, it is preferable that V and Y are each N. It is also preferable that W and Z are each S.

Preferably, there is provided a compound of formula (III): 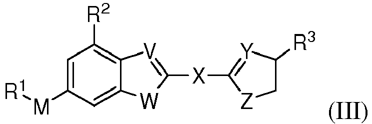 wherein
R¹ is hydrogen or optionally substituted alkyl (preferably hydrogen);
R² is a group independently selected from optionally substituted hydroxyl, optionally substituted amino, and optionally substituted alkyl;
R³ is optionally substituted C₁₋₆ alkyl carbonyloxy or carboxyl;
M is selected from O and S (preferably O);
V and Y are each independently selected from CR⁵ and N; and
W and Z are each independently selected from NR⁷, S, and O,
wherein R⁵ is a group selected from hydrogen, optionally substituted hydroxyl, optionally substituted alkyl, and optionally substituted amino, and
R⁷ is a group selected from hydrogen, optionally substituted alkyl, and optionally substituted aryl. Such compounds have been shown to possess an emission wavelength in the nIR, and therefore show improved spectral properties through haemoglobin, i.e. there is less interference and/or scatter when imaging is conducted *in vivo.* In addition, the compounds exhibit an excellent quantum yield, at least comparable to those known in the art, yet also provide an additional red-shift with Flue colour mutants. Furthermore, the compounds show good bioluminescence intensity *in vivo,* without requiring pre-adenylation.

Each of the optionally substituted groups of the compound of formula (II) and formula (III) may be substituted by:
(1) a group selected from -J-aryl, -J-heteroaryl, -J-heterocyclyl and -J-C₃₋₆ cycloalkyl, wherein J represents a bond or C₁₋₃ alkylene, and said aryl is selected from phenyl, said heteroaryl is selected from triazolyl, thiazolyl, thienyl, pyrazolyl, pyrimidyl, pyridazinyl, pyrazinyl, and pyridyl, said heterocyclyl is selected from pyrrolidinyl, azetidinyl, pyrazolidinyl, oxazolidinyl, piperidinyl, piperazinyl, morpholinyl, and thiazolidinyl, and said C₃₋₆ cycloalkyl is selected from cyclopropyl, cyclopentyl and cyclohexyl; or
(2) one to three substituents selected from
   (a) C₁₋₆ alkyl (preferably methyl, ethyl or isopropyl),
   (b) C₁₋₃ alkenyl (preferably propenyl),
   (c) halogen (preferably Cl or Br),
   (e) haloC₁₋₆ alkyl (preferably trifluoromethyl),
   (d) cyano,
   (e) amino, optionally mono-or di-substituted with C₁₋₃ alkyl, *tert*-butoxycarbonyl or benzyl,
   (f) C₁₋₃ alkoxy (preferably methoxy),
   (g) C₁₋₆ alkyl carbonyl (preferably acetyl),
   (h) C₁₋₃ alkoxy carbonyl,
   (i) C₁₋₃ alkyl carbonyloxy, including carboxyl, and
   (j) C₁₋₃ alkyl carbamoyl, including carbamoyl.

The compounds of the present invention may be in any stereoisomeric form. It is preferable, nevertheless, that the compounds possess the same stereoisomeric form as natural luciferin, i.e. *S*-stereochemistry at the α-position to the carbonyl functionality (on the basis of a dihydrothiazole ring).

In particular, the compound according to the invention may be selected from: 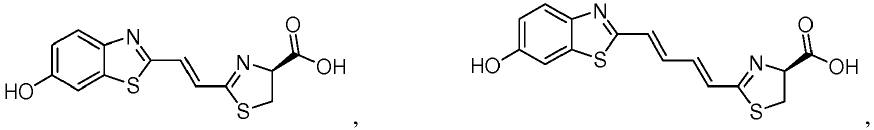 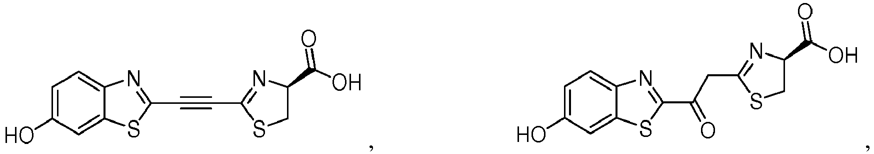 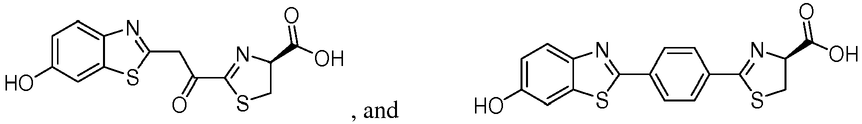 and C₁₋₆ alkyl esters thereof, e.g. methyl, ethyl, propyl, isopropyl, butyl, isobutyl and *tert-*butyl (preferably methyl and ethyl).

A compound according to the invention may be for use in *in vivo* imaging, preferably *in vivo* bioluminescence imaging. Furthermore, any of the preferred structural variants mentioned above in relation to formulae (II)-(III) also represent preferred aspects of this embodiment as *in vivo* imaging agents.

In particular, cells or animals bearing firefly luciferase may be detected using a number of common imaging devices. The most common form of such devices detects emitted photons using a lens focused onto a cooled charge-coupled device (CCD) with or without pre-intensification via a photomultiplier tube (e.g. IVIS imaging systems, by Perkin Elmer, MA, USA; and Photon Imager system, by Biospace Laboratories, Paris, France).

In addition, chemiluminescence reactions involving compounds according to the invention, or bioluminescence elicited using compounds according to the invention, and firefly luciferase in *in vitro* or diagnostic assays may be detected with common laboratory single tube or microplate luminometers, which employ either photomultiplier tube or CCD photon detectors (e.g. those sold by Promega, WI, USA).

In another aspect of the invention, there is provided a composition comprising a compound according to the invention, and one or more additional ingredients. Furthermore, any of the preferred structural variants mentioned above in relation to formulae (II)-(III) also represent preferred aspects of this composition.

The additional ingredients in the composition may include pharmaceutically acceptable diluents, excipients and carriers. Pharmaceutically acceptable diluents, excipients and carriers that may be used in the compositions include, but are not limited to, ion exchangers, alumina, aluminium stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulphate, disodium hydrogen phosphate, potassium hydrogen phosphate, sodium chloride, zinc salts, colloidal silica, magnesium trisilicate, polyvinyl pyrrolidone, coenzyme A, cellulose-based substances, polyethylene glycol, sodium carboxymethylcellulose, polyacrylates, waxes, polyethylene- polyoxypropylene-block polymers, polyethylene glycol and wool fat.

The composition of the invention may be administered orally, parenterally, by inhalation spray, topically, rectally, nasally, buccally, vaginally or via an implanted reservoir. Preferably, the compositions are administered parenterally, more specifically by injection. The composition may contain any conventional non-toxic pharmaceutically-acceptable carriers, adjuvants or vehicles. The term parenteral as used herein includes intraperitoneal, subcutaneous, intracutaneous, intravenous, intramuscular, intra-articular, intrasynovial, intrasternal, intrathecal, intraocular, intraorbital, intralesional and intracranial injection or infusion techniques. Preferably, the route of administration of the composition is intraperitoneal, intravenous or intramuscular administration (most preferably intraperitoneal).

The composition may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant such as Ph. Helv or a similar alcohol.

In another aspect of the invention, there is provided a use of the compound according to the invention as a bioluminescent marker for *in vitro* measurement of luciferase expression. Furthermore, any of the preferred structural variants mentioned above in relation to formulae (II)-(III) also represent preferred aspects of this use.

Given that the compound of the invention has specific utility as an *in vivo* imaging agent, there is also provided a method of *in vivo* imaging, comprising administering to a subject a compound according to the invention, and a luciferase enzyme or a polynucleotide encoding a luciferase enzyme, and measuring the emission resulting from the action of the luciferase enzyme on the compound. Furthermore, any of the preferred structural variants mentioned above in relation to formulae (II)-(III) also represent preferred aspects of this method.

The method may further comprising generating an image based on the measurements obtained.

In an additional aspect of the invention, there is provided a kit comprising a compound according to the invention, and a luciferase enzyme or a polynucleotide encoding a luciferase enzyme. Furthermore, any of the preferred structural variants mentioned above in relation to formulae (II)-(III) also represent preferred aspects of this kit.

The invention will now be described in more detail by way of example only, and with reference to the following figures.

### Description of the Figures

### Figure 1

Fluorescence spectra of compound 9 (often denoted 'infraluciferin' or 'iLH₂') in different pH conditions. The fluorescence spectra of compound 9 shows that it has redshifted absorbance, excitation and emission fluorescence spectra compared to native LH₂. In addition, higher pH further red-shifted the fluorescence excitation and emission, potentially indicating deprotonation and molecular conjugation in line with the resonance charge based mechanism. Importantly, compound 9 appeared to have emission maxima at different wavelengths dependent on the conditions.

### Figure 2

Bioluminescence spectra of native LH₂ and compound 9 ('analogue') with Flue colour mutants (x5 thermostable and x5 thermostable red). With a mutant that produces red emission with native LH₂ (λmax 620 nm), compound 9 produced a colour with λmax 700 nm, which is significantly more red-shifted than those in the art. Spectra experimental details: 50 µL of 5 µM Flue or red mutant in 100 mM tris-acetate, 10 mM magnesium sulfate and 2mM ethylenediaminetetraacetic acid (EDTA) (pH 7.8) was added to 20 µL 500 µM compound 9 with 70 µL PBS. Spectra were acquired in a Luminoskan Ascent machine (Thermo scientific, Waltham, MA, USA). Conversely, luciferin analogues known in the art do not show such a red-shift with Flue colour mutants.

### Figure 3

Light emission kinetics of compound 8 added to Raji cells expressing thermostable Flue. The methyl (Me) ester (compound 8) was seen to be enantio-stable, and was found to be active in mammalian and bacterial cells. The kinetics of saponification in mammalian cells showed a slow rise (ca. 2min) to maximal activity. Experimental details: 20 µl 1 mg/ml compound 8 in citrate buffer (pH 5) was added to 10 µl containing 1 x 10⁶ Raji cells expressing thermsotable Flue and light emission was recorded over 2 min in a Turner Designs TD20/20 instrument (Turner Designs, CA, USA).

### Figure 4

Relative light yield of compounds 8 and 12 *in vitro* compared to D-LH₂ ethyl ester measured in an intensified CCD-based photon imager with good sensitivity to red light. The difference in light yield from compound 8 was seen to be only ca. 150-times less than from LH₂ ethyl ester. Experimental details: 10 µL of ImM of the compounds was added to 50 µg pig liver esterase mixed with 5 mM ATP and 0.25 µM Flue enzymes. Light was captured in a Photon Imager machine (Biospace Lab, Paris, France).

### Figure 5

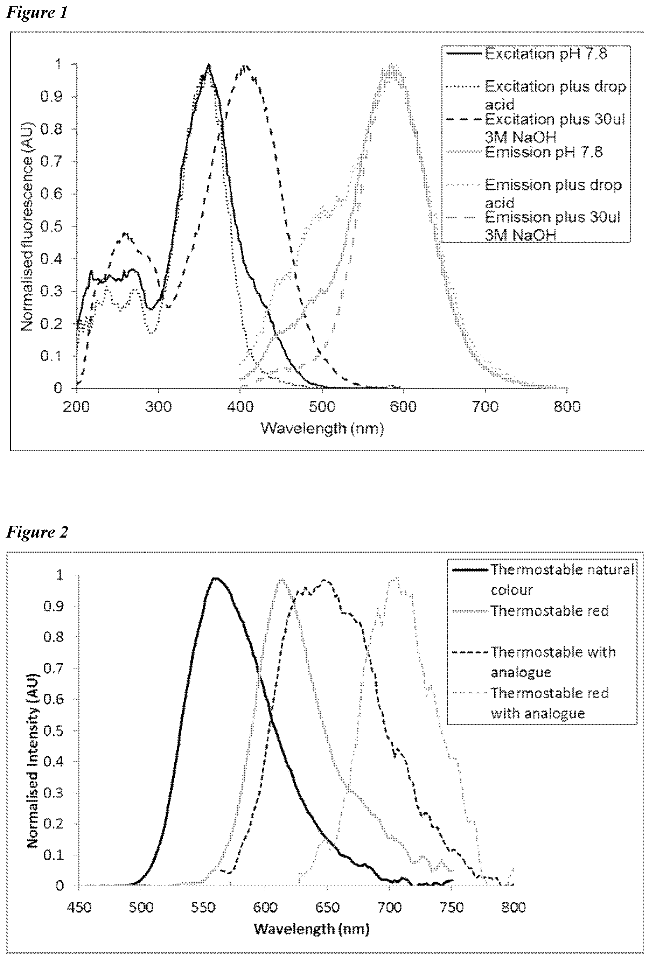

*In vitro* bioluminescence spectra of luciferase mutants x5 (left series) and x5 red (right series), with compound 9. *In vitro* bioluminescence spectra were acquired by imaging the samples through different filter sets and confirmed the two colours of compound 9 with x5 and x5 red. It may be possible to image both separately by applying 550-600nm bandpass and 830 longpass filters, respectively. Experimental details: 10 µg purified porcine liver esterase in TEM buffer was added to 1 mL of compound 8 dissolved in TEM buffer and incubated at 37° C for 15min before assay as per details in Figure 1. Light emission was captured in a Photon Imager device for 30s using different bandpass filters (Biospace Labs, Paris, France).

### Figure 6

Relative transmission of LH₂ ethyl ester and compound 12 through blood with natural and red coloured Flue mutants. When Raji cells expressing x5 Flue injected into mice containing systemic Raji cell lymphomas, the produced light that was imaged was ca. 8-times less in intensity than native LH₂ in the region of interest shown.

### Figure 7

Relative light yield of compound 12 (middle image) *in vivo* compared to D-LH₂ (right image) measured in an intensified CCD-based photon imager with good sensitivity to red light. The left image is a control with no filter and no substrate. The enhanced *in vivo* light yield compared to *in vitro* may be in part explained by the improved transmission of the emission of iLH₂ through blood compared to LH₂.

### Figure 8

Comparison of LH₂ ethyl ester (termed 'luciferin' in the figure) and compound 8 (termed 'infraluciferin' in the figure) light emission in a subcutaneous colon carcinoma model. In a xenograft model of colon carcinoma in nude mice (MFI NuNu), light from sub cutaneously engrafted LS274T cells WT Flue expressing mice imaged with iLH₂ ethyl ester was approximately 4 times less light than with compound 8. When subsequently imaged using an 830LP filter (infrared filter), the light yield from compound 8 was approximately 5-fold higher than from LH₂ ethyl ester. Experimental details: mice were inoculated with 3 x 10⁶ LS174T cells expressing WT Flue and 5 days later were injected with 5mg of LH₂ ethyl ester or compound 8 and imaged for a total of 30s in a Photon imager instrument (Biospace Labs, Paris, France). The infrared filter used was an 830 nm long pass (830 LP).

### Figure 9

Quantification of LH₂ ethyl ester and compound 8 light emission in a subcutaneous colon carcinoma model - no filter. Experimental details: M3 vision software (Biospace Labs, Paris, France) was used to process the light emission.

### Figure 10

Quantification of LH₂ ethyl ester and compound 8 light emission in a subcutaneous colon carcinoma model - 830LP filter. Experimental details: M3 vision software (Biospace Labs, Paris, France) was used to process the light emission.

### Figure 11

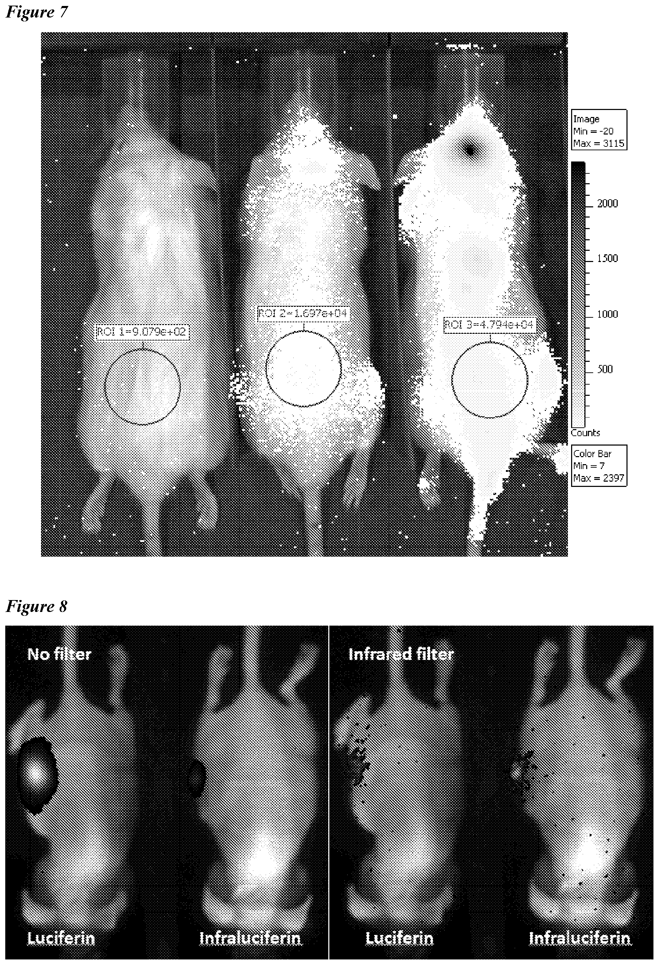

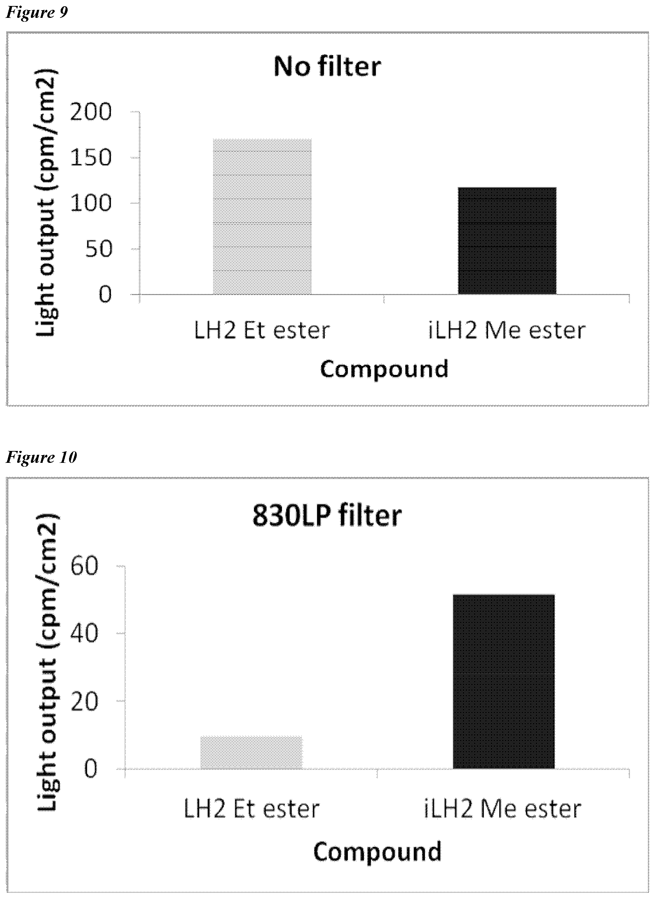

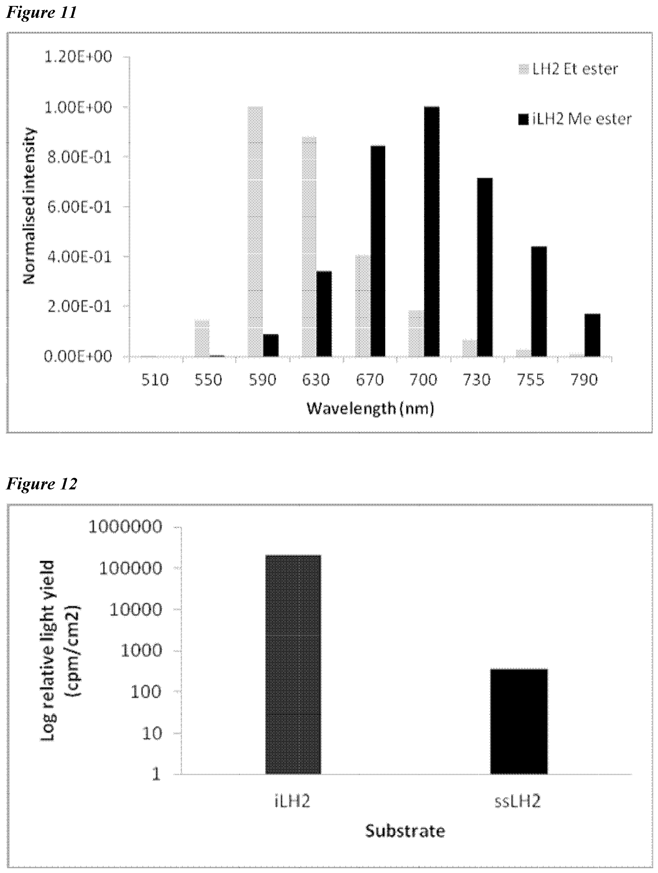

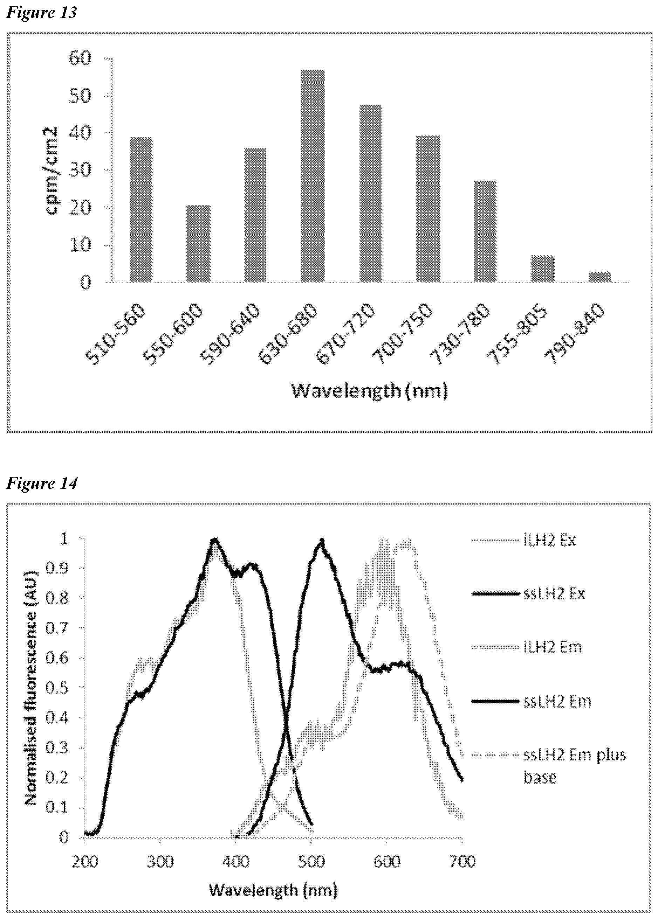

*In vivo* spectrum of light emission from LH₂ ethyl ester and compound 8. A larger proportion of light emitted with compound 8 *in vivo* was in the nIR portion of the electromagnetic spectrum than LH₂ ethyl ester. Experimental details as in Figure 8, but multiple acquisitions were acquired for 2 min each through a series of filters and corrected for light decay.

### Figure 12

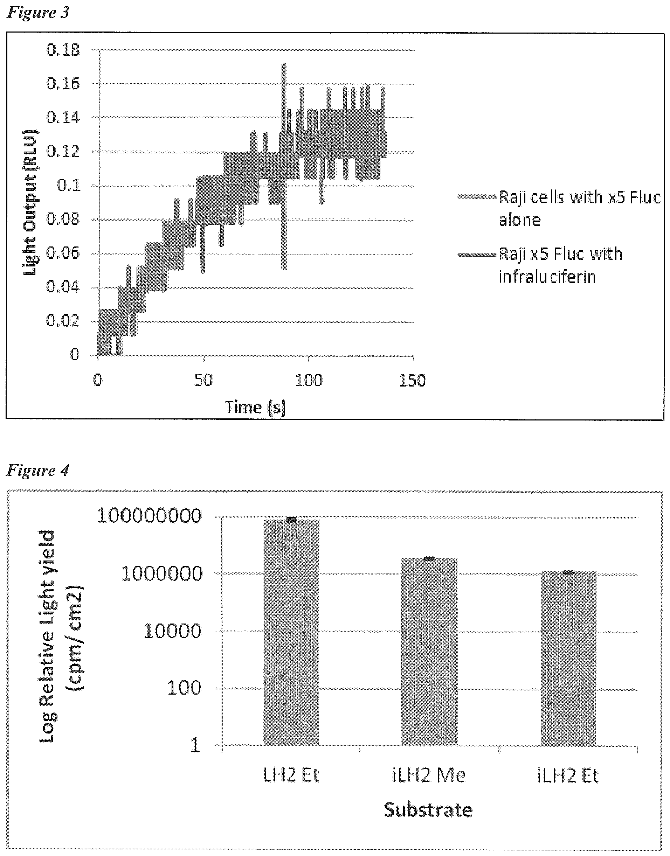

Light emission of compound 17 (denoted 'ssLH₂'). This compound was found to also generate light emission with purified x5 Flue, though this was less intense than emission with compound 8. Experimental details as in Figure 4.

### Figure 13

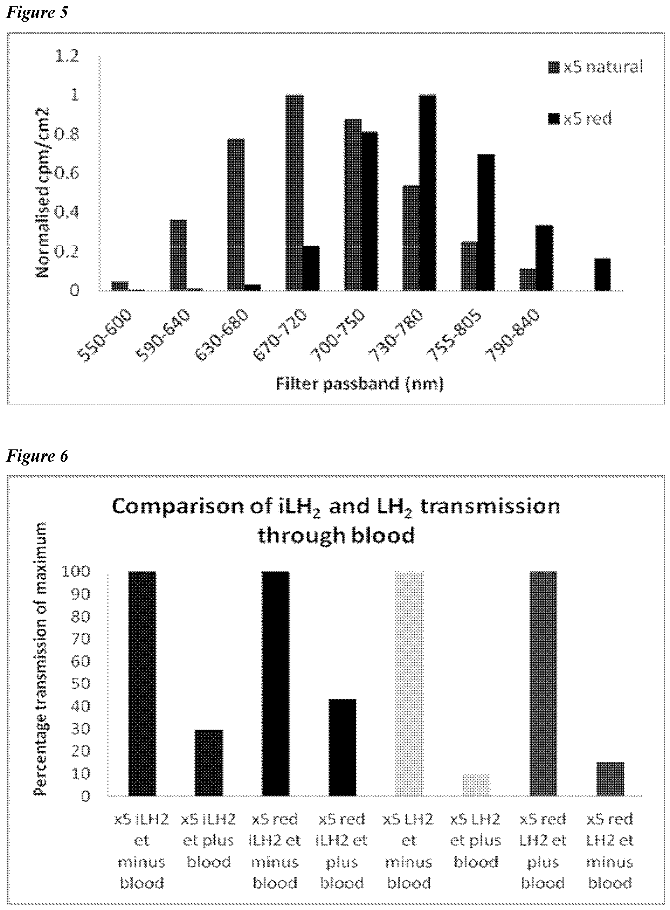

iCCD spectrum of x5 Flue with compound 17. The *in vitro* emission peaked between 630-680 nm. Experimental details as in figure 5.

### Figure 14

Fluorescence spectra of compound 8 against compound 17. The fluorescence spectra showed that compound 17 was further redshifted than compound 8 in its fluorescence excitation and emission properties, and also had access to two colour forms (pH dependent). Experimental details as in Figure 1.

### Examples

### 6-Hydroxy-2-(2-(4S-carboxy-4,5-dihydrothiazol-2-yl)ethenyl)benzothiazole (9)

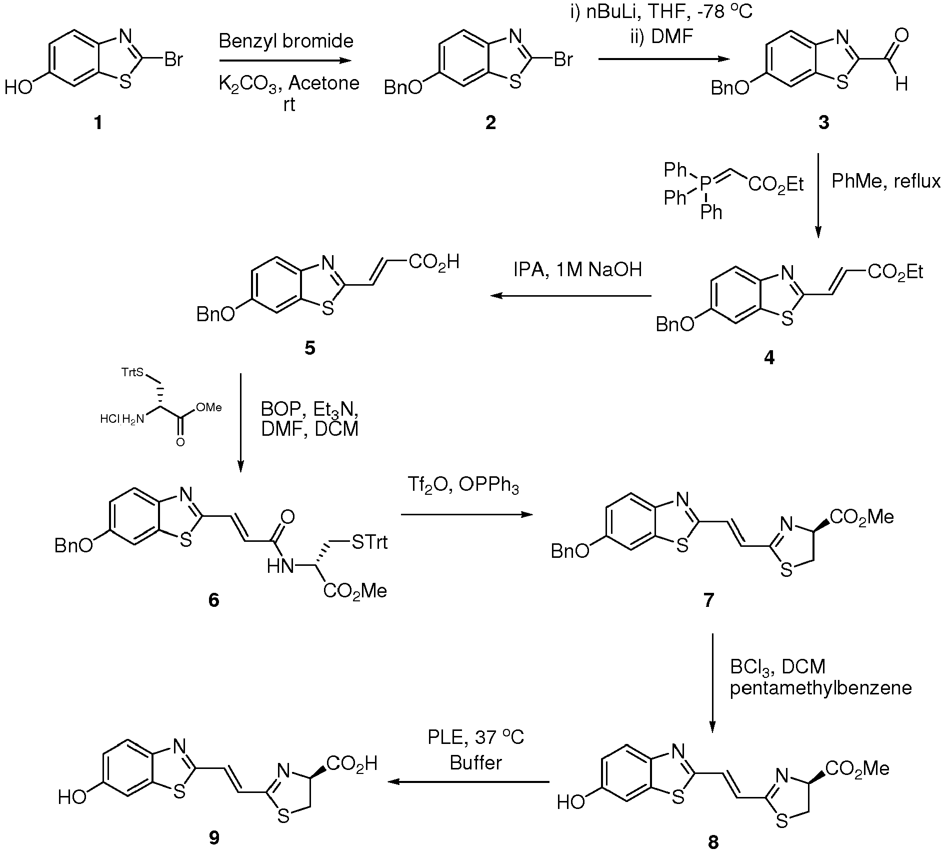

Amino acid, D-TrtCysOMe was synthesised using an analogous method to that developed for L-cysteine by Rudolph et al., J. Med. Chem. 2001, 44, 619 and protected as the methyl ester using thionyl chloride (Mays et al., Tetrahedron Lett., 2007, 48, 4579).

### 6-Benzoxy-2-bromo-benzothiazole (2)

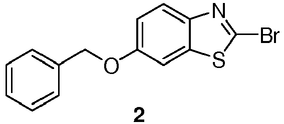

A solution of **1** (640 mg, 2.78 mmol) and BnBr (0.38 mL, 3.33 mmol) in acetone (50 mL) was treated with K₂CO₃ (1.09 g, 7.77 mmol) and stirred at rt for 16 h. After this time the reaction was filtered and concentrated *in vacuo.* Purification was achieved by flash column chromatography (5 % EtOAc/Pet. Ether) to give **2** (759 mg, 85 %) as a white solid, m.p. 58-60 °C; R_{f} = 0.61 (20 % EtOAc/Pet. Ether); IR νₘₐₓ 2937 (ν_{CH}), 2873 (ν_{CH}), 1598, 1557, 1483, 1452, 1380, 1282, 1246, 1222, 1210, 1016, 946 cm⁻¹; ¹H NMR (CDCl₃) δ 5.13 (2H, s, OC*H₂*Ar), 7.14 (1H, dd, *J* = 9.0, 2.6, Ar*H*), 7.33 (1H, d, *J* = 2.5, Ar*H*), 7.35-7.46 (6H, m, Ar*H*), 7.87 (1H, d, *J* = 8.9, Ar*H*); ¹³C NMR (CDCl₃) δ 70.8 (CH₂), 105.0 (CH), 116.5 (CH), 123.4 (CH), 127.5 (CH), 128.3 (CH), 128.8 (CH), 135.7 (C), 136.4 (C), 138.6 (C), 147.1 (C), 157.2 (C); m/z (ES+) 322 (100 %), 320 (100 %, M⁺+H), 321 (18 %), 272 (7 %), 272 (7 %); HRMS C₁₄H₁₁BrNOS calcd. 319.9745, found 319.9730; Anal. Calcd. for C₁₄H₁₀BrNOS: C, 52.51; H, 3.15; N, 4.37. Found C, 52.19; H, 3.03; N, 4.33 %.

### 6-Benzoxy-2-formyl-benzothiazole (3)

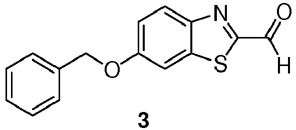

A solution of **2** (120 mg, 0.374 mmol) in THF (4 mL) was cooled to -78 °C, treated with ⁿBuLi (1.93 M, 0.22 mL, 0.412 mmol) (colour change from colourless to pale yellow) and stirred at -78 °C for 15 min. After this time DMF (0.12 mL, 1.55 mmol) was added dropwise and the resultant solution stirred at -78 °C for 1 h. The reaction was quenched with NaHCO_{3 (aq)} (2 mL), back extracted with EtOAc (2 × 10 mL), separated and concentrated *in vacuo.* Purification was achieved by flash column chromatography (5-10 % EtOAc/Pet. Ether) to give **3** (98 mg, 96 %) as a white solid, m.p. 112-114 °C; R_{f} = 0.51 (20 % EtOAc/Pet. Ether); IR νₘₐₓ 3035 (ν_{CH}), 2854 (ν_{CH}), 1677 (ν_{CO}), 1598, 1547, 1489, 1455, 1380, 1269, 1192, 1120, 999 cm⁻¹; ¹H NMR (CDCl₃) δ 5.19 (2H, s, OC*H*₂Ar), 7.30 (1H, dd, *J* = 9.1, 2.5, Ar*H*), 7.38-7.48 (7H, m, Ar*H*), 8.12 (1H, d, *J* = 9.1, Ar*H*), 10.1 (1H, s, C(O)*H*); ¹³C NMR (CDCl₃) δ 70.8 (CH₂), 105.0 (CH), 118.8 (CH), 126.7 (CH), 127.6 (CH), 128.5 (CH), 128.9 (CH), 136.0 (C), 138.6 (C), 148.5 (C), 159.5 (C), 163.2 (C), 185.2 (CH); m/z (ES+) 270 (100 %, M⁺+H), 210 (6 %), 132 (14 %, M⁺+H-C(O)H, OBn); HRMS C₁₅H₁₂NO₂S calcd. 270.0589, found 270.0585; Anal. Calcd. for C₁₅H₁₁NO₂S: C, 66.89; H, 4.12; N, 5.20. Found C, 66.62; H, 4.04; N, 5.10 %.

### (E)-3-(6-benzoxy-benzothiazol-2-yl)-acrylic acid ethyl ester (4)

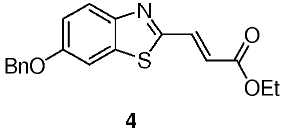

A solution of **3** (568 mg, 2.10 mmol) in PhMe (10 mL) was treated with (carbethoxymethylene)triphenylphosphorane (2.16 g, 6.31 mmol) and heated to reflux for 3 h. After this time the reaction was cooled to rt and concentrated *in vacuo.* Purification was achieved by flash column chromatography (10 % EtOAc/Pet. Ether) to give **4** (660 mg, 92 %) as a pale yellow solid. m.p 79-80 °C; R_{f} = 0.38 (20 % EtOAc/Pet. Ether); IR νₘₐₓ 3063 (ν_{CH}), 3038 (ν_{CH}), 2979 (ν_{CH}), 1703 (ν_{CO}) 1595, 1551, 1488, 1445, 1386, 1368, 1254, 1106, 1018, 958 cm⁻¹; 1.36 (3H, t, *J* = 7.1, CH₂C*H₃*), 4.31 (2H, q, *J* = 7.1, C*H*₂CH₃), 5.16 (2H, s, OC*H₂*Ar), 6.71 (1H, d, *J* = 15.9, CHC(O)OEt), 7.20 (1H, dd, *J* = 9.0, 2.5, Ar*H*), 7.35-7.48 (6H, m, Ar*H*), 7.85 (1H, d, *J* = 15.9, C*H*C(N)S), 7.96 (1H, d, *J* = 9.0, Ar*H*); ¹³C NMR (CDCl₃) δ 14.3 (CH₃), 61.2 (CH₂), 70.7 (CH₂), 105.2 (CH), 117.3 (CH), 124.7 (CH), 125.1 (CH), 127.6 (CH), 128.3 (CH), 128.8 (CH), 136.4 (C), 136.8 (C), 137.0 (CH), 148.7 (C), 158.0 (C), 161.2 (C), 165.8 (C); m/z (ES+) 340 (100%, M⁺+H), 339 (7%, M⁺), 294 (7%, M⁺+H-OEt), 91 (7%, PhCH₂); HRMS C₁₉H₁₈NO₃S calcd. 340.1007, found 340.1003; Anal. Calcd. for C₁₉H₁₇NO₃S: C, 67.24; H, 5.05; N, 4.13. Found C, 67.04; H, 5.02; N, 4.09 %.

### (E)-3-(6-benzoxy-benzothiazol-2-yl)-acrylic acid (5)

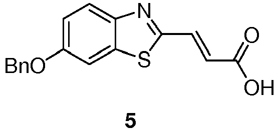

A suspension of **4** (500 mg, 1.47 mmol) in ⁱPrOH (18.3 mL) was treated with NaOH (4.70 mL, 1.0 M) and stirred at rt overnight. An additional 10 mL of H₂O was added so that all precipitate had dissolved and the resultant solution acidified to pH 1 with HCl (1 M). The yellow precipitate was isolated by filtration and washed with H₂O (30 mL) to give **5** (458 mg, quant.) as a fine yellow powder. m.p 174-176 °C; R_{f} = 0.40 (10 % MeOH/DCM); IR νₘₐₓ 3031 (ν_{CH}), 2866 (ν_{CH}), 2543 (ν_{OH}), 1694 (ν_{CO}), 1599, 1558, 1497, 1451, 1312, 1259, 1205, 1051, 1013, 966 cm⁻¹; ¹H NMR (DMSO) δ 15.20 (2H, s, OC*H₂*Ar), 6.72 (1H, d, *J* = 15.9, C*H*C(O)OEt), 7.23 (1H, dd, *J* = 9.0, 2.5, Ar*H*), 7.32-7.50 (5H, m, Ar*H*), 7.69 (1H, d, *J* = 15.9, C*H*C(N)S), 7.83 (1H, d, *J* = 2.4, Ar*H*), 7.97 (1H, d, *J* = 9.0, Ar*H*); ¹³C NMR (DMSO) δ 69.9 (CH₂), 105.8 (CH), 117.2 (CH), 124.2 (CH), 126.0 (CH), 127.9 (CH), 128.0 (CH), 128.5 (CH), 135.9 (CH), 136.6 (C), 136.6 (C), 147.9 (C), 157.4 (C), 160.8 (C), 166.4 (C); m/z (CI) 402 (26%), 312 (100%, M⁺+H), 294 (11%, M⁺+H-OH), 91 (5%, PhCH₂); HRMS C₁₇H₁₄NO₃S calcd. 312.0694, found 312.0691.

### D-(Methyl 2-((E)-3-(6-benzoxybenzothiazol-2-yl)acrylamido)-3-(tritylthio)proponoate (6)

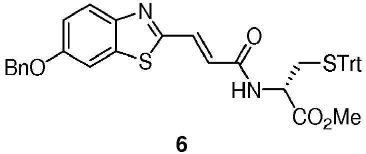

A solution of **5** (134 mg, 0.424 mmol) in DMF (4.2 mL) was treated with Et₃N (70 µL, 1.02 mmol) and cooled to 0 °C. The solution was treated with a solution of aminoacid (192 mg, 0.508 mmol) in DCM (2 mL) followed by a solution of BOP (231 mg, 0.508 mmol) in DCM (2.2 mL) and the resultant solution stirred at 0 °C for 2 h. After this time the reaction mixture was quenched with saturated NaHCO_{3(aq)} (2 mL) and taken up in EtOAc (10 mL), the aqueous layer was back extracted using EtOAc (2 × 5 mL), organics dried over MgSO₄, filtered and concentrated *in vacuo.* Purification was achieved by flash column chromatography (20 % EtOAc/Pet. Ether) to give **6** (220 mg, 80 %) as a yellow solid. m.p. 83-86 °C; R_{f} = 0.28 (30 % EtOAc/Pet. Ether); [α]_{D} +62.6 (c 0.95, CHCl₃); CSP HPLC analysis (Chiracel OD-H (150 × 4.6 mm), eluent: hexane: ⁱPrOH, 70:30, flow 0.5 mL/min, 14 bar) determined > 98 % ee [t_{R} (minor) = 43.07 min, t_{R} (major) = 26.59 min]; IR νₘₐₓ 1741 (vco), 1662, 1627, 1596, 1488, 1446, 1346, 1319, 1260, 1196, 1051, 1001, 966 cm⁻¹; ¹H NMR (CDCl₃) δ 2.73 (1H, dd, *J* = 12.7, 4.6, C*H*₂STrt), 2.82 (1H, dd, *J* = 12.7, 5.6, C*H*₂STrt), 3.76 (3H, s, OC*H₃*), 4.76 (1H, dt, *J* = 7.8, 5.2, C*H*CH₂), 5.17 (2H, s, OC*H₂*Ph), 6.09 (1H, d, *J* = 7.8, N*H*), 6.65 (1H, d, *J* = 15.5, C*H*C(O)N), 7.20-7.49 (22H, m, Ar*H*), 7.73 (1H, d, *J* = 15.5, C*H*C(N)S), 7.96 (1H, d, *J* = 9.0, Ar*H*); ¹³C NMR (CDCl₃) δ 33.8 (CH₂), 51.4 (CH), 52.9 (CH₃), 67.2 (C), 70.7 (CH₂), 105.2 (CH), 117.2 (CH), 124.6 (CH), 126.8 (CH), 127.1 (CH), 127.6 (CH), 128.1 (CH), 128.3 (CH), 128.8 (CH), 129.6 (CH), 134.2 (CH), 136.4 (C), 144.3 (C), 154.2 (C), 157.9 (C), 161.4 (C), 163.8 (C), 170.7 (C); m/z (ES+) 693 (96%, M⁺+Na), 595 (9%), 244 (18%), 243 (100%, M⁺- STrt,CO₂Me, Bn, H), 228 (8%), 165 (22%); HRMS C₄₀H₃₄N₂O₄NaS₂ calcd. 693.1858, found 693.1855.

### 6-Benzoxy-2-(2-(4S-methoxycarbonyl-4,5-dihydrothiazol-2-yl)ethenyl)benzothiazole (7)

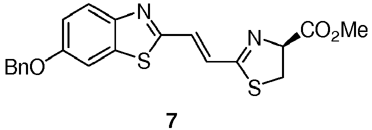

A solution of Ph₃PO (386 mg, 1.40 mmol) in DCM (10 mL) was cooled to 0 °C in an ice bath and treated with Tf₂O (0.160 mL, 0.874 mmol) added dropwise over 5 min. The resultant solution was stirred at 0 °C for 30 min and a solution of **6** (220 mg, 0.329 mmol) in DCM (4 mL) was then added dropwise over 5 min. The reaction was stirred at 0 °C for 10 min and then quenched with phosphate buffer (4 mL). Extracted into DCM (3 × 10 mL) and separated. The organic layer was dried over MgSO₄, filtered and concentrated *in vacuo.* Purification was achieved by flash column chromatography (20-40 % EtOAc/Pet. Ether) to give **7** (88 mg, 65 %) as a pale yellow solid. m.p. 98-100 °C; R_{f} = 0.32 (40 % EtOAc/Pet. Ether); IR νₘₐₓ 3032, 2947 (ν_{CH}), 1726 (ν_{CO}), 1599, 1569, 1557, 1480, 1454, 1431, 1380, 1319, 1261, 1242, 1225, 1199, 1176, 1043, 1018, 946 cm⁻¹; ¹H NMR (CDCl₃) δ 3.63 (1H, dd, *J* = 10.9, 9.3, C*H*₂S), 3.71 (1H, dd, *J* = 11.0, 9.3, C*H*₂S), 3.86 (3H, s, OC*H₃*), 5.15 (2H, s, OC*H*₂Ph), 5.26 (1H, t, *J* = 9.3, C*H*CH₂), 7.18 (1H, dd, *J* = 9.0, 2.5, Ar*H*), 7.31-7.48 (8H, m, ArH, 2 × C*H*C(N)S), 7.93 (1H, d, *J* = 9.0, Ar*H*); ¹³C NMR (CDCl₃) δ 35.1 (CH₂), 53.1 (CH₃), 70.7 (CH₂), 78.2 (CH), 105.2 (CH), 117.2 (CH), 124.6 (CH), 127.6 (CH), 128.4 (CH), 128.8 (CH), 129.0 (CH), 135.2 (CH), 136.4 (C), 136.7 (C), 148.7 (C), 158.0 (C), 162.0 (C), 169.4 (C), 170.9 (C); m/z (ES+) 474 (32%), 411 (100%, M⁺+H), 320 (12%); HRMS C₂₁H₁₉N₂O₃S₂ calcd. 411.0837, found 411.0831; Anal. Calcd. for C₂₁H₁₈N₂O₃S₂: C, 61.44; H, 4.42; N, 6.82. Found C, 61.38; H, 4.34; N, 6.74 %.

CSP HPLC analysis determined 45-95 % ee dependant on scale of reaction and purity of Tf₂O. Optical purity could be achieved by dissolving **7** in MeCN and concentrating *in vacuo* until a precipitate appeared. Filtration and concentration of the filtrate gave **7.** [α]_{D} -28.4 (c 0.91, CHCl₃); CSP HPLC analysis (Chiracel OD-H, eluent: hexane: ⁱPrOH, 70:30, flow 0.5 mL/min, 14 bar) > 98 % ee [t_{R} (minor) = 21.8 min, t_{R} (major) = 30.7 min].

### 6-Hydroxy-2-(2-(4S-methoxycarbonyl-4,5-dihydrothiazol-2-yl)ethenyl)benzothiazole (8)

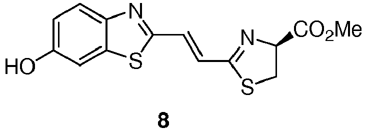

A solution of **7** (55 mg, 0.133 mmol) in DCM (2 mL) was cooled to -78 °C in an acetone/CO₂₍ₛ₎ bath and treated with pentamethylbenzene (110 mg, 0.580 mmol) followed by BCl₃ (0.39 mL, 0.399 mmol, 1 M in DCM) added dropwise over 5 min. The reaction was stirred at -78 °C for 20 min and then quenched with phosphate buffer (2 mL). Extracted into DCM (3 × 5 mL) and separated. The organic layer was dried over MgSO₄, filtered and concentrated *in vacuo.* Purification was achieved by flash column chromatography (40-100 % EtOAc/Pet. Ether) to give **8** (34 mg, 79 %, 98 % b.r.s.m) as a pale yellow solid. m.p. 163-165 °C; R_{f} = 0.13 (40 % EtOAc/Pet. Ether); IR νₘₐₓ 3210 (ν_{OH}), 3043 (ν_{CH}), 2954, 1731 (ν_{CO}), 1632, 1595, 1558, 1480, 1435, 1314, 1199, 1116, 1022, 949 cm⁻¹; ¹H NMR (MeOD) δ 3.70 (1H, dd, *J* = 20.1, 11.2, C*H*₂S), 3.72 (1H, dd, *J* = 20.8, 11.2, C*H*₂S), 3.81 (3H, s, OC*H₃*), 5.33 (1H, t, *J* = 9.0, C*H*CO₂Me), 7.03 (1H, dd, *J* = 8.9, 2.5, Ar*H*), 7.29 (1H, d, *J* = 16.1, C*H*C(N)S), 7.31 (1H, d, *J* = 2.3, Ar*H*), 7.41 (1H, d, *J* = 16.1, C*H*C(N)S), 7.82 (1H, d, *J* = 8.9, Ar*H*); ¹³C NMR (MeOD) δ 35.7 (CH₂), 53.2 (CH₃), 79.0 (CH), 107.4 (CH), 118.1 (CH), 125.1 (CH), 128.9 (CH), 136.0 (CH), 138.1 (C), 148.6 (C), 158.5 (C), 162.2 (C), 171.6 (C), 172.2 (C).

CSP HPLC analysis determined 65-90 % ee dependant on scale of reaction and purity of BCl₃. Optical purity could be achieved by dissolving **8** in MeCN and concentrating *in vacuo* until a precipitate appeared. Filtration and concentration of the filtrate gave **8**. CSP HPLC analysis (Chiracel AD, eluent: hexane: ⁱPrOH, 70:30, flow 0.5 mL/min, 23 bar) > 98 % ee [t_{R} (major) = 6.35 min, t_{R} (minor) = 9.90 min].

### 6-Hydroxy-2-(2-(4S-carboxy-4,5-dihydrothiazol-2-yl)ethenyl)benzothiazole (9)

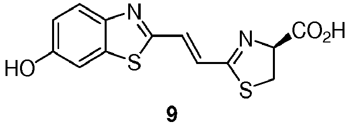

A solution of **8** in phosphate buffer (1 mg/mL) was treated with PLE (5 µg per 1 mg of **8**) and incubated at 37 °C for 24 h. After this time the saponified ester 9 was used directly without isolation.

### Synthesis of 6-Hydroxy-2-(2-(4S-ethoxycarbonyl-4,5-dihydrothiazol-2-yl)ethenyl) benzothiazole (12)

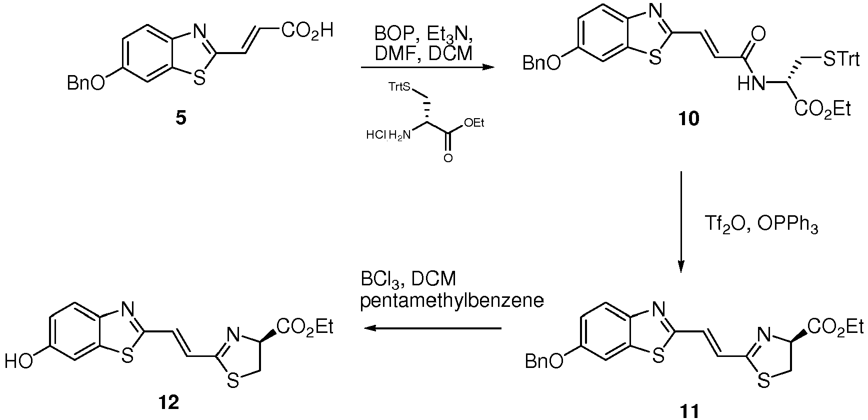

### D-(Ethyl 2-((E)-3-(6-benzoxybenzothiazol-2-yl)acrylamido)- 3-(tritylthio)proponoate (10)

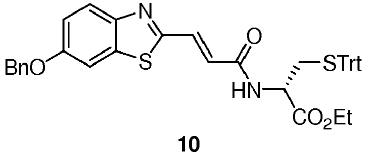

A solution of **5** (239 mg, 0.755 mmol) in DMF (7.6 mL) was treated with Et₃N (0.124 mL, 1.82 mmol) and cooled to 0 °C. The solution was treated with a solution of aminoacid (354 mg, 0.908 mmol) in DCM (3 mL) followed by a solution of BOP (414 mg, 0.908 mmol) in DCM (4.6 mL) and the resultant solution stirred at 0 °C for 2 h. After this time the reaction mixture was quenched with saturated NaHCO_{3(aq)} (5 mL) and taken up in EtOAc (20 mL), the aqueous layer was back extracted using EtOAc (2 × 10 mL), organics dried over MgSO₄, filtered and concentrated *in vacuo.* Purification was achieved by flash column chromatography (20 % EtOAc/Pet. Ether) to give **10** (422 mg, 82 %) as an orange solid. m.p. 75-78 °C; R_{f} = 0.23 (30 % EtOAc/Pet. Ether); [α]_{D} +82.0 (c 1.05, CHCl₃); CSP HPLC analysis (Chiracel AD (300 × 4.6 mm), eluent: hexane: ⁱPrOH, 60:40, flow 1 mL/min, 26 bar) determined > 99 % ee [t_{R} (minor) = 16.52 min, t_{R} (major) = 9.67 min]; IR νₘₐₓ 1735 (ν_{CO}), 1667, 1628, 1596, 1488, 1446, 1371, 1344, 1260, 1189, 1050, 1019, 966 cm⁻¹; ¹H NMR (CDCl₃) δ 1.27 (3H, t, *J* = 7.2, CH₂C*H₃*), 2.70 (1H, dd, *J* = 12.6, 4.5, C*H*₂STrt), 2.80 (1H, dd, *J* = 12.6, 5.5, C*H*₂STrt), 4.22 (2H, m, C*H*₂CH₃), 4.75 (1H, dt, *J* = 7.7, 4.8, C*H*CH₂), 5.17 (2H, s, OC*H₂*Ph), 6.14 (1H, d, *J* = 7.8, NH), 6.68 (1H, d, *J* = 15.5, CHC(O)N), 7.20-7.49 (22H, m, Ar*H*), 7.74 (1H, d, *J* = 15.5, C*H*C(N)S), 7.96 (1H, d, *J* = 9.0, Ar*H*); ¹³C NMR (CDCl₃) δ 14.3 (CH₃), 34.0 (CH₂), 51.5 (CH), 62.2 (CH₂), 67.1 (C), 70.7 (CH₂), 105.2 (CH), 117.2 (CH), 124.6 (CH), 126.9 (CH), 127.1 (CH), 127.6 (CH), 128.2 (CH), 128.4 (CH), 128.9 (CH), 129.6 (CH), 134.2 (CH), 136.4 (C), 136.8 (C), 144.4 (C), 148.8 (C), 157.9 (C), 161.5 (C), 163.8 (C), 170.2 (C); m/z (ES+) 707 (100%, M⁺+Na), 685 (5%, M⁺+H), 338 (27%, M⁺+H-STrt, CO₂Et), 243 (100%), 228 (23%), 165 (59%); HRMS C₄₁H₃₆NaN₂O₄S₂ calcd. 707.2014, found 707.2039; Anal. Calcd. for C₄₁H₃₆N₂O₄S₂: C, 71.90; H, 5.30; N, 4.09. Found C, 71.43; H, 5.30; N, 3.98 %.

### 6-Benzoxy-2-(2-(4S-ethoxycarbonyl-4,5-dihydrothiazol-2-yl)ethenyl)benzothiazole (11)

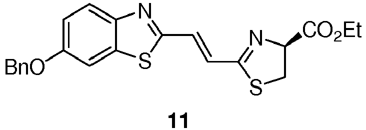

A solution of Ph₃PO (955 mg, 3.45 mmol) in DCM (26 mL) was cooled to 0 °C in an ice bath and treated with Tf₂O (0.39 mL, 2.16 mmol) added dropwise over 5 min. The resultant solution was stirred at 0 °C for 30 min and a solution of **10** (544 mg, 0.797 mmol) in DCM (8 mL) was then added dropwise over 5 min. The reaction was stirred at 0 °C for 10 min and then quenched with phosphate buffer (10 mL). Extracted into DCM (3 × 15 mL) and separated. The organic layer was dried over MgSO₄, filtered and concentrated *in vacuo.* Purification was achieved by flash column chromatography (20-40 % EtOAc/Pet. Ether) to give **11** (250 mg, 74 %) as a pale yellow solid. m.p. 114-117 °C; R_{f} = 0.39 (40 % EtOAc/Pet. Ether); IR νₘₐₓ 2977 (ν_{CH}), 1729 (ν_{CO}), 1597, 1555, 1479, 1453, 1379, 1316, 1261, 1222, 1199, 1049, 1014, 947 cm⁻¹; ¹H NMR (CDCl₃) δ 1.36 (3H, t, *J* = 6.6, CH₂C*H₃*), 3.63 (1H, dd, *J* = 11.0, 9.4, C*H*₂S), 3.68 (1H, dd, *J* = 11.0, 9.4, C*H*₂S), 4.27-4.38 (2H, m, C*H₂*CH₃), 5.16 (2H, s, OC*H₂*Ar), 5.24 (1H, t, *J* = 9.3, C*H*CO₂Et), 7.19 (1H, dd, *J* = 9.0, 2.5, Ar*H*), 7.30-7.49 (8H, m, Ar*H*), 7.93 (1H, d, *J* = 9.0, Ar*H*); ¹³C NMR (CDCl₃) δ 14.3 (CH₃), 35.1 (CH₂), 62.2 (CH₂), 70.7 (CH₂), 78.5 (CH), 105.2 (CH), 117.2 (CH), 124.6 (CH), 127.6 (CH), 128.4 (CH), 128.8 (CH), 129.2 (CH), 135.0 (CH), 136.4 (C), 136.6 (C), 148.7 (C), 158.0 (C), 162.1 (C), 169.2 (C), 170.5 (C); m/z (ES+) 425 (100%, M⁺+H), 334 (25%, M⁺-Bn), 293 (11%); HRMS C₂₂H₂₁N₂O₃S₂ calcd. 425.0994, found 425.0998.

CSP HPLC analysis determined 82-92 % ee dependant on scale of reaction and purity of Tf₂O. Optical purity could be achieved by dissolving **11** in MeCN and concentrating *in vacuo* until a precipitate appeared. Filtration and concentration of the filtrate gave **11.** CSP HPLC analysis (Chiracel AD, eluent: hexane: ⁱPrOH, 60:40, flow 1 mL/min, 26 bar) determined > 98 % ee [t_{R} (minor) = 23.60 min, t_{R} (major) = 11.19 min].

### 6-Hydroxy-2-(2-(4S-ethoxycarbonyl-4,5-dihydrothiazol-2-yl)ethenyl)benzothiazole (12)

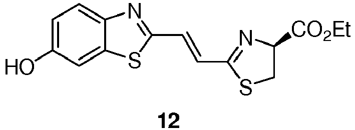

A solution of **11** (67 mg, 0.158 mmol) in DCM (2.7 mL) was cooled to -78 °C in an acetone/CO₂₍ₛ₎ bath and treated with pentamethylbenzne (130 mg, 0.788 mmol) followed by BCl₃ (0.46 mL, 0.472 mmol, 1 M in DCM) added dropwise over 5 min. The reaction was stirred at -78 °C for 20 min and then quenched with phosphate buffer (2 mL). Extracted into DCM (3 × 5 mL) and separated. The organic layer was dried over MgSO₄, filtered and concentrated *in vacuo.* Purification was achieved by flash column chromatography (40-100 % EtOAc/Pet. Ether) to give **12** (38 mg, 72 %, 96 % b.r.s.m) as a pale yellow solid. m.p. 155-157 °C; R_{f} = 0.11 (40 % EtOAc/Pet. Ether); IR νₘₐₓ 3377 (ν_{OH}), 2983 (ν_{CH}), 1732 (ν_{CO}) 1592, 1568, 1476, 1449, 1379, 1369, 1321, 1281, 1254, 1192, 1116, 1062, 1027, 944 cm⁻¹; ¹H NMR (CDCl₃) δ 1.36 (3H, t, *J* = 7.1, CH₂C*H₃*), 3.64 (1H, dd, *J* = 20.5, 10.8, C*H*₂S), 3.68 (1H, dd, *J* = 20.3, 10.8, C*H*₂S), 4.30-4.36 (2H, m, C*H₂*CH₃), 5.25 (1H, t, *J* = 9.2, C*H*CO₂Et), 5.76 (1H, s, O*H*), 7.03 (1H, dd, *J* = 8.8, 2.4, Ar*H*), 7.28 (1H, d, J = 16.1, C*H*C(N)S) 7.30 (1H, d, *J* = 2.4, Ar*H*), 7.38 (1H, d, *J* = 16.1, C*H*C(N)S), 7.89 (1H, d, J= 8.8, Ar*H*); ¹³C NMR (CDCl₃) δ 14.3 (CH₃), 35.1 (CH₂), 62.3 (CH₂), 78.4 (CH), 107.0 (CH), 116.7 (CH), 124.7 (CH), 129.1 (CH), 135.0 (CH), 136.7 (C), 148.6 (C), 155.0 (C), 162.0 (C), 169.5 (C), 170.6 (C); m/z (ES+) 335 (100%, M⁺+H), 307 (12%, M⁺+H-Et), 261 (48%, M⁺+H-CO₂Et), 203 (47 %, M⁺+H-SCH₂CHCO₂Et); HRMS C₁₅H₁₅N₂O₃S₂ calcd. 335.0524, found 335.0526.

CSP HPLC analysis determined 80-92 % ee dependant on scale of reaction and purity of BCl₃. Optical purity could be achieved by dissolving **12** in MeCN and concentrating *in vacuo* until a precipitate appeared. Filtration and concentration of the filtrate gave **12.** CSP HPLC analysis (Chiracel AD, eluent: hexane: ⁱPrOH, 70:30, flow 1.0 mL/min, 23 bar) > 98 % ee [t_{R} (major) = 6.85 min, t_{R} (minor) = 9.30 min].

### Synthesis of 6-Hydroxy-2-(4-1E,3E-(4S-ethoxycarbonyl-4,5-dihydrothiazol-2-yl)buta-2,4-dienyl)benzothiazole (17)

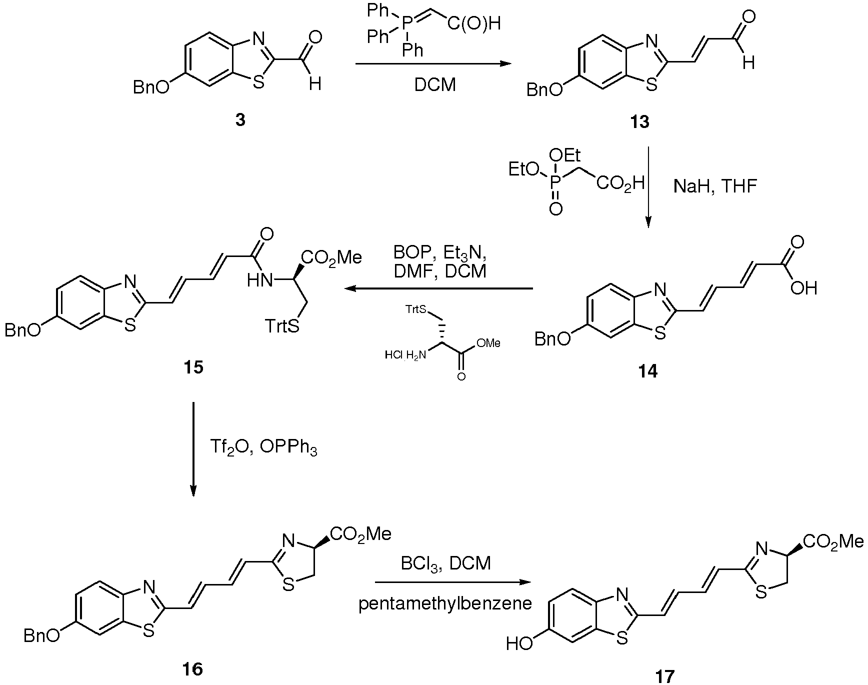

### (E)-3-(6-Benzoxv-benzothiazol-2-yl)-prop-2-enal (13)

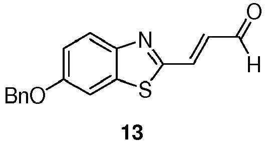

A solution of **3** (100 mg, 0.370 mmol) in DCM (2 mL) was treated with (triphenylphosphorarylidene)acetaldehyde (124 mg, 0.407 mmol) and stirred at rt for 2 h. The reaction was quenched with saturated NaHCO_{3(aq)} (2 mL), separated and back extracted using DCM (2 × 5 mL). The organic layers were dried over MgSO₄, filtered and concentrated *in vacuo.* Purification was achieved by flash column chromatography (5 % EtOAc/Pet. Ether) to give **13** (76 mg, 69 %) as a yellow solid.
m.p. 124-126 °C; R_{f} = 0.27 (20 % EtOAc/Pet. Ether); IR νₘₐₓ 1674 (ν_{CO}), 1589, 1555, 1481, 1464, 1389, 1264, 1245, 1224, 1201, 1112, 1095, 1052, 1003, 992, 966 cm⁻¹; ¹H NMR (CDCl₃) δ 5.17 (2H, s, OC*H₂*Ar), 6.89 (1H, dd, *J* = 16.0, 7.6, CHC(O)H), 7.23 (1H, dd, *J* = 9.0, 2.5, Ar*H*), 7.34-7.50 (6H, m, Ar*H*), 7.72 (1H, d, *J* = 16.0, C*H*C(N)S), 8.01 (1H, d, *J* = 9.0, Ar*H*), 9.79 (1H, d, *J* = 7.2, C(O)H); ¹³C NMR (CDCl₃) δ 70.8 (CH₂), 105.0 (CH), 117.8 (CH), 125.2 (CH), 127.6 (CH), 128.4 (CH), 128.9 (CH), 133.5 (CH), 136.2 (C), 137.4 (C), 143.8 (CH), 148.9 (C), 158.5 (C), 160.8 (C), 192.6 (CH); m/z (CI) 296 (100%, M⁺+H), 220 (21%, M⁺+2H-Ph), 217 (12%, M⁺-H, Ph); HRMS C₁₇H₁₄NO₂S calcd. 296.0745, found 296.0743.

### (E)-5-(6-Benzoxy-benzothiazol-2-yl)-2,4-pentadienoic acid (14)

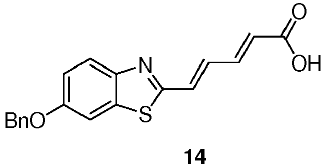

A solution of diethylphosphonoacetic acid (80 mg, 0.411 mmol) in THF (4 mL) was cooled to 0 °C and treated with NaH (8 mg, 0.333 mmol, 95 %), gas evolution. The solution was stirred at 0 °C for 15 min and then treated with a solution of **13** (122 mg, 0.411 mmol) in THF (1 mL), added dropwise. The resultant red solution was stirred at 0 °C for 30 min and then allowed to warm to rt over 1 h. After this time the solution was concentrated, taken up in H₂O, acidified (1 M HCl) and the resultant brown precipitate filtered to give give **14** (119 mg, 86 %) as a brown solid. m.p. 226-228 °C; R_{f} = 0.18 (5 % MeOH/DCM); IR νₘₐₓ 1678 (ν_{CO}), 1610, 1594, 1553, 1482, 1449, 1362, 1321, 1269, 1241, 1208, 1181, 1149, 1058, 1021, 1002, 935 cm⁻¹; ¹H NMR (DMSO) δ 5.19 (2H, s, OC*H₂*Ar), 6.28 (1H, d, *J* = 14.9, C*H*C(O)OH), 7.20 (1H, dd, *J* = 8.9, 2.5, Ar*H*), 7.32-7.50 (8H, m, 3×C*H*CH, 5×Ar*H*), 7.79 (1H, d, *J* = 2.5, Ar*H*), 8.01 (1H, d, *J* = 8.9, Ar*H*); ¹³C NMR (DMSO) δ 69.9 (CH₂), 105.9 (CH), 116.8 (CH), 123.7 (CH), 126.3 (CH), 128.0 (CH), 128.1 (CH), 128.5 (CH), 131.9 (CH), 133.9 (CH), 136.1 (C), 136.7 (C), 142.3 (CH), 148.1 (C), 157.0 (C), 162.8 (C), 167.3 (C); m/z (ES⁺) 338 (100%, M⁺+H), 320 (15%, M⁺-OH), 247 (19%, M⁺-H, Ph); HRMS C₁₉H₁₆NO₃S calcd. 338.0851, found 338.0845.

### Methyl 2-((2E,4E-5-(6-Benzoxy-benzothiazol-2-yl)penta-2,4-dienoylamido)-3-(tritylthio) propononate (15)

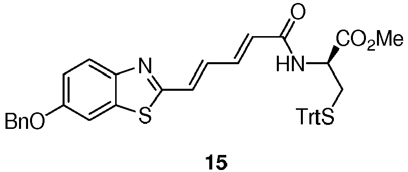

A solution of **14** (118 mg, 0.350 mmol) in DMF (3.5 mL) was treated with Et₃N (58 µL, 0.841 mmol) and cooled to 0 °C. The solution was treated with a solution of aminoacid (158 mg, 0.419 mmol) in DCM (1.5 mL) followed by a solution of BOP (191 mg, 0.419 mmol) in DCM (2.0 mL) and the resultant solution warmed to rt and stirred for 16 h. After this time the reaction mixture was quenched with saturated NaHCO_{3(aq)} (5 mL), separated and the aqueous layer was back extracted using DCM (2 × 10 mL), organics dried over MgSO₄, filtered and concentrated *in vacuo.* Purification was achieved by flash column chromatography (20 % EtOAc/Pet. Ether) to give **15** (128 mg, 53 %) as an orange oil. R_{f} = 0.40 (40 % EtOAc/Pet. Ether); [α]_{D} +56.3 (c 1.00, CHCl₃); IR νₘₐₓ 3057 (ν_{CH}), 1734 (ν_{CO}), 1659, 1597, 1555, 1485, 1444, 1317, 1244, 1259, 1203, 1177, 1051, 996 cm⁻¹; ¹H NMR (CDCl₃) δ 2.71 (1H, dd, *J* = 12.6, 4.7, C*H*₂S), 2.76 (1H, dd, *J* = 12.6, 5.5, C*H*₂S), 3.74 (3H, s, OC*H₃*), 4.68 (1H, dt, *J* = 7.6, 5.2, C*H*CO₂Me), 5.16 (2H, s, OC*H₂*Ar), 6.05 (1H, d, *J* = 7.7, C(O)N*H*), 6.09 (1H, d, *J* = 14.9, C*H*C(O)NH), 7.10-7.50 (25H, m, 3×C*H*CH, 22×Ar*H*), 7.92 (1H, d, *J* = 8.9, Ar*H*); ¹³C NMR (CDCl₃) δ 33.9 (CH₂), 51.3 (CH₃), 52.8 (CH), 67.1 (C), 70.7 (CH₂), 105.3 (CH), 116.8 (CH), 124.1 (CH), 126.9 (CH), 127.0 (CH), 127.6 (CH), 128.1 (CH), 128.3 (CH), 128.8 (CH), 129.6 (CH), 131.9 (CH0, 133.2 (CH), 136.3 (C), 136.5 (C), 139.9 (CH), 144.3 (C), 148.7 (C), 157.6 (C), 163.1 (C), 164.7 (C), 170.9 (C); m/z (ES⁺) 719 (100%, M⁺+Na), 697 (12%, M⁺+H), 643 (10%), 244 (22%, M⁺-Bn, C(CO₂Me)(CH₂STrt))), 243 (78%, M⁺-H, Bn, C(CO₂Me)(CH₂STrt))), 228 (12%, Trt), 165 (32%); HRMS C₄₂H₃₆N₂O₄NaS₂ calcd. 719.2014, found 719.1990.

### 6-Benzoxy-2-(4-1E,3E-(4S-methoxycarbonyl-4,5-dihydrothiazol-2-yl)buta-2,4-dienyl) benzothiazole (16)

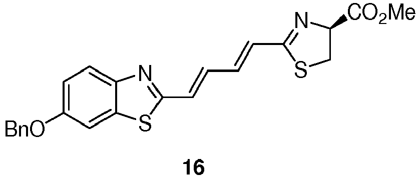

A solution of Ph₃PO (217 mg, 0.784 mmol) in DCM (6.0 mL) was cooled to 0 °C in an ice bath and treated with Tf₂O (89 µL, 0.491 mmol) added dropwise over 5 min. The resultant solution was stirred at 0 °C for 30 min and a solution of **15** (127 mg, 0.182 mmol) in DCM (2.5 mL) was then added dropwise over 5 min. The reaction was stirred at 0 °C for 20 min and then quenched with phosphate buffer (10 mL). Extracted into DCM (3 × 10 mL) and separated. The organic layer was dried over MgSO₄, filtered and concentrated *in vacuo.* Purification was achieved by flash column chromatography (20-40 % EtOAc/Pet. Ether) to give 16 (41 mg, 52 %) as a yellow solid. m.p. 104-107 °C; R_{f} = 0.36 (40 % EtOAc/Pet. Ether); IR νₘₐₓ 3032 (ν_{CH}), 2949 (ν_{CH}), 1739 (ν_{CO}), 1596, 1560, 1484, 1448, 1260, 1232, 1204, 1173, 1053, 983cm⁻¹; ¹H NMR (CDCl₃) δ 3.58 (1H, dd, *J* = 11.0, 9.3, C*H*₂S), 3.65 (1H, dd, *J* = 11.0, 9.3, C*H*₂S), 3.84 (3H, s, OC*H₃*), 5.14, (2H, s, OC*H₂*Ar), 5.21 (1H, t, *J* = 9.3, 5.2, C*H*CO₂Me), 6.78 (1H, d, *J* = 15.4, C*H*C(N)S), 6.97 (1H, dd, *J* = 15.4, 10.7, C CHCHC(N)S), 7.04 (1H, d, *J* = 15.5, C*H*C(N)S), 7.13-7.49 (8H, m, 1×C*H*CH, 7×Ar*H*), 7.90 (1H, d, *J* = 8.9, Ar*H*); ¹³C NMR (CDCl₃) δ 34.9 (CH₂), 53.1 (CH₃), 70.7 (CH), 78.1 (CH₂), 105.3 (CH), 116.9 (CH), 124.1 (CH), 126.9 (CH), 127.6 (CH), 128.8 (CH), 129.9 (CH), 134.0 (CH), 136.3 (C), 136.5 (C), 140.3 (CH), 148.7 (C), 157.6 (C), 163.1 (C), 169.6 (C), 171.1 (C); m/z (ES⁺) 459 (25%, M⁺+Na), 437 (100%, M⁺+H), 342 (42%, M⁺-Bn, 4H), 279 (93%,), 243 (39%), 196 (22%), 165 (25%); HRMS C₂₃H₂₁N₂O₃S₂ calcd. 437.0994, found 437.0989.

### 6-Hydroxy-2-(4-1E,3E-(4S-methoxycarbonyl-4,5-dihydrothiazol-2-yl)buta-2,4-dienyl) benzothiazole (17)

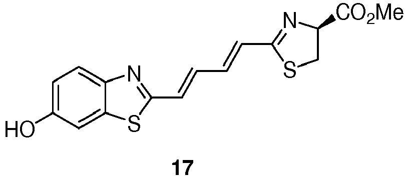

A solution of **16** (40 mg, 0.0916 mmol) in DCM (1.5 mL) was cooled to -78 °C in an acetone/CO₂₍ₛ₎ bath and treated with pentamethylbenzne (75 mg, 0.456 mmol) followed by BCl₃ (0.27 mL, 0.275 mmol, 1 M in DCM) added dropwise over 5 min. The reaction was stirred at -78 °C for 20 min and then quenched with phosphate buffer (2 mL). Extracted into DCM (3 × 5 mL) and separated. The organic layer was dried over MgSO₄, filtered and concentrated *in vacuo.* Purification was achieved by flash column chromatography (30-60 % EtOAc/Pet. Ether) to give **17** (17 mg, 54 %, 79 % b.r.s.m) as a pale yellow solid. m.p. 156-158 °C; R_{f} = 0.20 (40 % EtOAc/Pet. Ether); IR νₘₐₓ 3210 (ν_{OH}), 3046 (ν_{CH}), 2920, 2853, 1731 (ν_{CO}), 1631, 1595, 1557, 1480, 1435, 1314, 1199, 1116, 1022, 949 cm⁻¹; ¹H NMR (MeOD) δ 3.64 (1H, dd, *J* = 19.9, 11.2, C*H*₂S), 3.66 (1H, dd, *J* = 22.5, 11.2, C*H*₂S), 3.81 (3H, s, OC*H₃*), 5.27 (1H, t, *J* = 9.0, C*H*CO₂Me), 6.78 (1H, d, *J* = 15.4, C*H*C(N)S), 6.99 (1H, dd, *J* = 15.4, 2.4, CHCHC(N)S), 7.08-7.18 (2H, m, CHC(N)S, Ar*H*), 7.25-7.31 (1H, m, C*H*C(N)S), 7.29 (1H, d, *J* = 2.4, Ar*H*), 7.76 (1H, d, *J* = 8.9, Ar*H*); ¹³C NMR (MeOD) δ 35.3 (CH₂), 53.1 (CH₃), 78.6 (CH), 107.4 (CH), 117.7 (CH), 124.5 (CH), 129.3 (CH), 131.9 (CH), 135.5 (CH), 142.2 (CH), 148.4 (C), 158.1 (C), 164.2 (C), 172.3 (C), 172.6 (C); m/z (ES+) 347 (100%, M⁺+H), 279 (39%), 229 (15%), 202 (28%), 196 (58%); HRMS C₁₆H₁₅N₂O₃S₂ calcd. 347.0524, found 347.0536.

### Synthesis of 2-(4-(6-hydroxybenzothiazol-2-yl)aryl)-4,5-dihydro-thiazole-4-carboxylic acid (20)

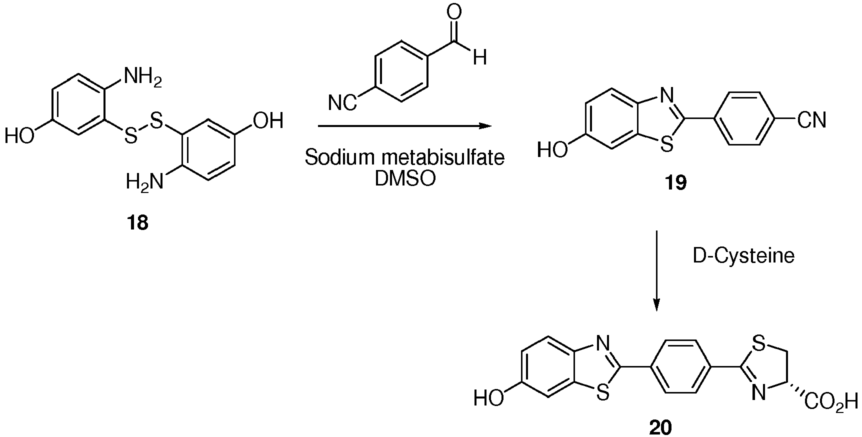

The synthesis may begin with commercially available 2-amino-6-methoxybenzothiazole. The amine may then be converted into the bridged thiol (1**8)** using KOH following the method described by Fink et al., Bioorg & Med Chem Lett, 2006, 16, 1532.

### 4-(6-hydroxybenzothiazol-2-yl)benzonitrile (18)

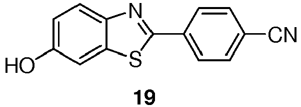

A solution of **18** (104 mg, 0.371 mmol) and 4-cyanobenzaldehyde (99 mg, 0.757 mmol) in anhydrous DMSO (3 mL) was treated with sodium metabisulfate and heated to 140 °C for 3 h. After this time the reaction was cooled to rt and H₂O added (10 mL). The resultant brown precipitate was filtered giving **19** (55 mg, 29 %) as a brown solid. R_{f} = 0.51 (40 % EtOAc/Pet. Ether); ¹H NMR (MeOD) δ 7.04 (1H, dd, *J* = 10.9, 2.3, Ar*H*), 7.36 (1H, d, *J* = 2.3, Ar*H*), 7.88 (2H, d, *J* = 8.4, Ar*H*), 7.88 (1H, d, Ar*H*), 8.19 (2H, d, *J* = 8.4, Ar*H*); ¹³C NMR (MeOD) δ 107.5 (CH), 114.7 (C), 117.9 (CH), 119.3 (C), 125.1 (CH), 128.6 (CH), 134.1 (CH), 138.2 (C), 138.9 (C), 148.9 (C), 158.1 (C), 163.6 (C).

### Synthesis of 6-hydroxy-2-(1-keto-2-(4S-methoxycarbonyl-4,5-dihydrothiazol-2-yl)ethyl) benzothiazole (24)

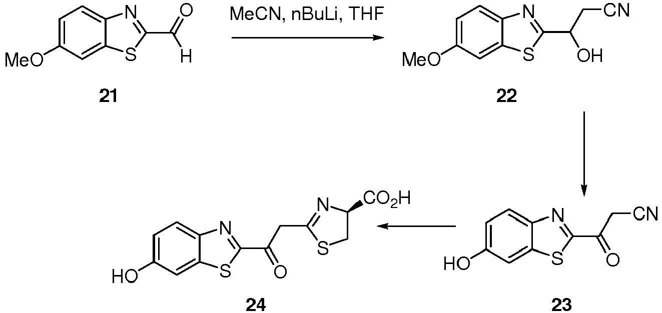

The synthesis may begin with commercially available 6-methoxy-2-methylbenzothiazole. The methyl group may then be oxidised using SeO₂ following the method described in US patent US4826833.

### 2-Hydroxy-3-(6-methoxy-benzothiazol-2-yl)-propionitrile (22)

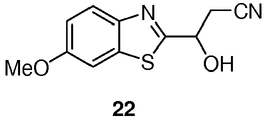

THF (5 mL) was cooled to -78 °C using an acetone/CO₂₍ₛ₎ bath and nBuLi (0.39 mL, 0.621 mmol, 1.6 M in hexanes) was added dropwise. The resultant solution was stirred for 5 min and then treated with MeCN (34 µL, 0.650 mmol), stirred 15 min and a solution of **21** (100 mg, 0.518 mmol) in THF (2 mL) was added dropwise. The reaction mixture was removed from the /CO₂₍ₛ₎ bath and stirred for 2 h. After this time the reaction was quenched using saturated NaHCO_{3(aq)} (2 mL), extracted with EtOAc (10 mL), washed H₂O (5 mL), dried (MgSO₄), filtered and concentrated *in vacuo.* Purification was achieved by flash column chromatography (50 % EtOAc/Pet. Ether) to give **22** (51 mg, 42 %) as an orange solid. m.p. 78-80 °C; R_{f} = 0.32 (50 % EtOAc/Pet. Ether); IR νₘₐₓ 3209 (ν_{OH}), 2013 (ν_{CN}), 1605 (ν_{CO}), 1559, 1519, 1469, 1436, 1262, 1227, 1091, 1056, 1025 cm⁻¹; ¹H NMR (CDCl₃) δ 3.30 (1H, dd, *J* = 16.8, 7.3, C*H*₂CN), 3.20 (1H, dd, *J* = 16.8, 4.4, C*H*₂CN), 3.70 (1H, d, *J* = 5.7, O*H*), 3.90 (3H, s, O*CH₃*), 5.38 (1H, app dd, *J* = 5.8, 10.7, C*H*OH), 7.12 (1H, dd, *J* = 8.9, 2.5, Ar*H*), 7.36 (1H, *J* = 2.4, Ar*H*), 7.88 (1H, *J* = 9.0, Ar*H*); ¹³C NMR (CDCl₃) δ 26.8 (CH₂), 56.0 (CH), 68.2 (CH), 104.2 (CH), 116.2 (CH), 116.7 (C), 123.9 (CH), 136.6 (C), 147.2 (C), 158.1 (C), 168.3 (C); m/z (EI⁺) 234 (27%, M⁺), 194 (100%, M⁺-CH₂CN), 166 (10%, M⁺-CH(OH)CH₂CN), 151 (29 %), 123 (14 %, M⁺-)Me, CCH(OH)CH₂CN), 95 (7%); HRMS C₁₁H₁₀N₂O₂S calcd. 234.0458, found 234.0459.

Oxidation and deprotection of **22** may then be achieved to provide **23**, and **23** may be converted to **24** by manipulation of the nitrile group and condensation with cysteine.

### Synthesis of 6-hydroxy-2-(2-(4S-methoxycarbonyl-4,5-dihydrothiazol-2-yl)ethynyl) benzothiazole (26)

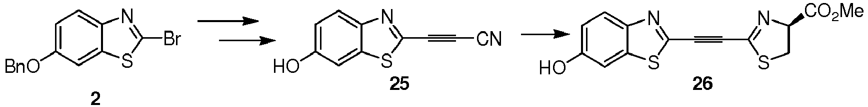

Sonagashira coupling of **2** and ethynyltrimethylsilane, and subsequent (hydolysis and reaction with phenyl cyanate may provide nitrile **25**. Ths may then be converted to **26** by manipulation of the nitrile andcondensation with cysteine.

### Bioluminescence characterisation of compounds

### Polynucleotides encoding mutant luciferases

x5 luciferase:
x5 red:

### Bioluminescence

Table of bioluminescence emission wavelength maximum (λmax) with native LH₂ and compound 9.

| **Name of mutant** | **λmax with luciferin (nm)** | **λmax with compound 9(nm)** |
|---|---|---|
| WT Flue | 557 | 662 |
| x5 Fluc | 557 | 638 |
| x5 red Flue | 620 | 700 |

Bioluminescence λmax values for LH₂ and compound 9 were tested with purified enzymes. Experimental details as in Figure 2. These results show that compound 9 represents a near infrared (nIR) multiparametric luciferin analogue.

### Saponification kinetics

Apparent kinetic parameters for LH₂ ethyl ester and compound 12.

| **Substrate** | ***Apparent* Km (µM)** | ***Apparent* Kcat (RLU s⁻¹) (x 10¹⁵)** | ***Apparent* Kcat/Km (s⁻¹ µM⁻¹) (x 10¹⁵)** |
|---|---|---|---|
| LH₂ ethyl ester | 11.9±0.6 | 861+206 | 75±18 |
| Compound 12 | 11.8+1.9 | 17.5+9 | 1.5+1 |

*Michaelis-Menten kinetics:* concentrations of each ester between 1.9mM and 0.38mM were incubated with 100µg of pig liver esterase at 37°C for 15min and allowed to come to room temperature before the initiation of light by addition of 10pmol Flue enzyme and 5.3mM ATP in 55µl reactions. Light was captured using the Photon Imager instrument (Biospace Labs, Paris, France) and the Hanes-Woolf plot was used to analyse kinetic parameters. PMT temperature varied between -9-12°C.

The kinetics of saponification in mammalian cells showed a slow rise (ca. 2min) to maximal activity. The apparent Km of compound 12 was seen to be the same as LH₂ indicating similar affinity for the substrate, although the Kcat was lower, possibly due to emitter quenching.

## Claims

1. A compound according to the formula (II): 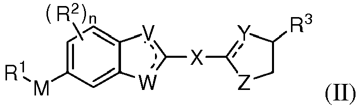 wherein
R¹ is hydrogen or an optionally substituted alkyl group;
each R², when present, is a group independently selected from optionally substituted hydroxyl, optionally substituted amino, optionally substituted thiol, optionally substituted alkyl, and optionally substituted aryl;
R³ is a carboxyl group or an optionally substituted C₁₋₆ alkyl carbonyloxy group;
M is a group selected from O, S, and NR⁴, wherein R⁴ is a group selected from hydrogen, optionally substituted alkyl, optionally substituted alkenyl, optionally substituted aryl, optionally substituted heteroaryl, and optionally substituted heterocyclyl;
X is a group selected from optionally substituted alkenylene, optionally substituted alkynylene, optionally substituted arylene, and combinations thereof, or optionally substituted alkylene carbonyl;
n is 0, 1, 2, or 3;
V and Y are each independently selected from CR⁵ and N;
W and Z are each independently selected from NR⁷, S, and O;
R⁵ is a group selected from hydrogen, optionally substituted hydroxyl, optionally substituted alkyl, and optionally substituted amino; and
R⁷ is a group selected from hydrogen, optionally substituted alkyl, and optionally substituted aryl;
wherein each of the optionally substituted groups listed above is optionally substituted by:
(1) a group selected from -J-aryl, -J-heteroaryl, -J-heterocyclyl and -J-C₃₋₆ cycloalkyl, wherein J represents a bond or C₁₋₃ alkylene, and said aryl is selected from phenyl, said heteroaryl is selected from triazolyl, thiazolyl, thienyl, pyrazolyl, pyrimidyl, pyridazinyl, pyrazinyl, and pyridyl, said heterocyclyl is selected from pyrrolidinyl, azetidinyl, pyrazolidinyl, oxazolidinyl, piperidinyl, piperazinyl, morpholinyl, and thiazolidinyl, and said C₃₋₆ cycloalkyl is selected from cyclopropyl, cyclopentyl and cyclohexyl; or
(2) one to three substituents selected from
(a) C₁₋₆ alkyl (preferably methyl, ethyl or isopropyl),
(b) C₁₋₃ alkenyl (preferably propenyl),
(c) halogen (preferably Cl or Br),
(e) haloC₁₋₆ alkyl (preferably trifluoromethyl),
(d) cyano,
(e) amino, optionally mono-or di-substituted with C₁₋₃ alkyl, *tert*-butoxycarbonyl or benzyl,
(f) C₁₋₃ alkoxy (preferably methoxy),
(g) C₁₋₆ alkyl carbonyl (preferably acetyl),
(h) C₁₋₃ alkoxy carbonyl,
(i) C₁₋₃ alkyl carbonyloxy, including carboxyl, and
(j) C₁₋₃ alkyl carbamoyl, including carbamoyl.

2. A compound according to claim 1, wherein n is 0 or 1.

3. A compound according to claim 1 or claim 2, having the formula (III): 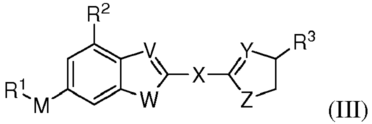 wherein
V and Y are each independently selected from CR⁵ and N; and
W and Z are each independently selected from NR⁷, S, and O.

4. A compound according to any preceding claim, wherein V and Y are each N, and/or wherein W and Z are each S.

5. A compound according to any preceding claim, wherein R¹ is hydrogen, and/or
wherein R² is a group selected from optionally substituted hydroxyl, optionally substituted amino, and optionally substituted alkyl, and/or
wherein X is a group selected from optionally substituted alkenylene, optionally substituted alkynylene, and optionally substituted alkylene carbonyl, preferably wherein X is an optionally substituted alkenylene group; and/or wherein M is O;
wherein the optional substituents of each of the optionally substituted groups are as defined in claim 1.

6. A compound according to any preceding claim selected from 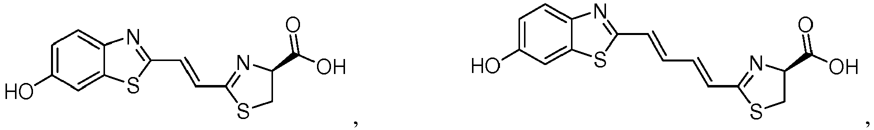 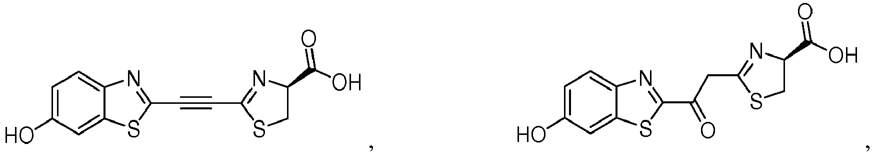 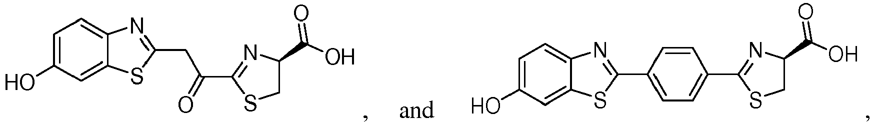 and C₁₋₆ alkyl esters thereof.

7. A compound according to any preceding claim for use in *in vivo* imaging.

8. A composition comprising a compound according to any one of claims 1 to 6, and one or more additional ingredients.

9. Use of the compound according to any one of claims 1 to 6 as a bioluminescent marker for *in vitro* measurement of luciferase expression.

10. A method of *in vivo* imaging, comprising
administering to a subject a compound according to any one of claims 1 to 6 and a luciferase enzyme or a polynucleotide encoding a luciferase enzyme, and
measuring the emission resulting from the action of the luciferase enzyme on the compound.

11. A method according to claim 10, further comprising generating an image based on the measurements obtained.

12. A kit comprising a compound according to any one of claims 1 to 6, and a luciferase enzyme or a polynucleotide encoding a luciferase enzyme.

## Patentansprüche

1. Verbindung gemäß der Formel (II): 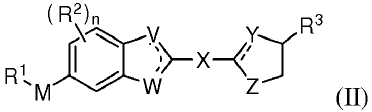 wobei
R¹ Wasserstoff oder eine wahlweise substituierte Alkyl-Gruppe ist;
jeder R², wenn vorhanden, eine Gruppe ist, die unabhängig voneinander aus wahlweise substituiertem Hydroxyl, wahlweise substituiertem Amino, wahlweise substituiertem Thiol, wahlweise substituiertem Alkyl und wahlweise substituiertem Aryl ausgewählt ist;
R³ eine Carboxyl-Gruppe oder eine wahlweise substituierte C₁₋₆-Alkylcarbonyloxy-Gruppe ist;
M eine Gruppe ist, die aus O, S und NR⁴ ausgewählt ist, wobei R⁴ eine Gruppe ist, die aus Wasserstoff, wahlweise substituiertem Alkyl, wahlweise substituiertem Alkenyl, wahlweise substituiertem Aryl, wahlweise substituiertem Heteroaryl und wahlweise substituiertem Heterocyclyl ausgewählt ist;
X eine Gruppe ist, die aus wahlweise substituiertem Alkenylen, wahlweise substituiertem Alkynylen, wahlweise substituiertem Arylen und Kombinationen davon oder wahlweise substituiertem Alkylencarbonyl ausgewählt ist;
n 0, 1, 2 oder 3 ist;
V und Y jeweils unabhängig voneinander aus CR⁵ und N ausgewählt sind;
W und Z jeweils unabhängig voneinander aus NR⁷, S und O ausgewählt sind;
R⁵ eine Gruppe ist, die aus Wasserstoff, wahlweise substituiertem Hydroxyl, wahlweise substituiertem Alkyl und wahlweise substituiertem Amino ausgewählt ist; und
R⁷ eine Gruppe ist, die aus Wasserstoff, wahlweise substituiertem Alkyl und wahlweise substituiertem Aryl ausgewählt ist;
wobei jede der oben genannten wahlweise substituierten Gruppen wahlweise substituiert ist mit:
(1) einer Gruppe, die aus -J-Aryl, -J-Heteroaryl, -J-Heterocyclyl und -J-C₃₋₆-Cycloalkyl ausgewählt ist, wobei J eine Bindung oder C₁₋₃-Alkylen darstellt, und das Aryl aus Phenyl ausgewählt ist, das Heteroaryl aus Triazolyl, Thiazolyl, Thienyl, Pyrazolyl, Pyrimidyl, Pyridazinyl, Pyrazinyl und Pyridyl ausgewählt ist, das Heterocyclyl aus Pyrrolidinyl, Azetidinyl, Pyrazolidinyl, Oxazolidinyl, Piperidinyl, Piperazinyl, Morpholinyl und Thiazolidinyl ausgewählt ist und das C₃₋₆-Cycloalkyl aus Cyclopropyl, Cyclopentyl und Cyclohexyl ausgewäht ist; oder
(2) einem bis drei Substituenten, die aus
(a) C₁₋₆-Alkyl (vorzugsweise Methly, Ethyl oder Isopropyl),
(b) C₁₋₃-Alkenyl (vorzugsweise Propenyl),
(c) Halogen (vorzugsweise Cl oder Br),
(e) Halogen-C₁₋₆-Alkyl (vorzugsweise Trifluormethyl),
(d) Cyano,
(e) Amino, wahlweise mono- oder di-substituiert mit C₁₋₃-Alkyl, *tert-*Butoxycarbonyl oder Benzyl,
(f) C₁₋₃-Alkoxy (vorzugsweise Methoxy),
(g) C₁₋₆-Alkylcarbonyl (vorzugsweise Acetyl),
(h) C₁₋₃-Alkoxycarbonyl,
(i) C₁₋₃-Alkylcarbonyloxy einschließlich Carboxyl und
(j) C₁₋₃-Alkylcarbamoyl einschließlich Carbamoyl ausgewählt sind.

2. Verbindung gemäß Anspruch 1, wobei n 0 oder 1 ist.

3. Verbindung nach Anspruch 1 oder Anspruch 2, die die Formel (III) hat: 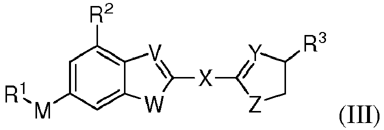 wobei
V und Y jeweils unabhängig voneinander aus CR⁵ und N ausgewählt sind; und
W und Z jeweils unabhängig voneinander aus NR⁷, S und O ausgewählt sind.

4. Verbindung gemäß einem vorhergehenden Anspruch, wobei V und Y jeweils N sind und/oder wobei W und Z jeweils S sind.

5. Verbindung gemäß einem vorhergehenden Anspruch, wobei R¹ Wasserstoff ist und/oder wobei R² eine Gruppe ist, die aus wahlweise substituiertem Hydroxyl, wahlweise substituiertem Amino und wahlweise substituiertem Alkyl ausgewählt ist und/oder
wobei X eine Gruppe ist, die aus wahlweise substituiertem Alkenylen, wahlweise substituiertem Alkynylen und wahlweise substituiertem Alkylencarbonyl ausgewählt ist,
wobei X vorzugsweise eine wahlweise substituierte Alkenylen-Gruppe ist; und/oder wobei M O ist;
wobei die wahlweisen Substituenten jeder wahlweise substituierten Gruppe wie in Anspruch 1 definiert sind.

6. Verbindung gemäß einem vorhergehenden Anspruch ausgewählt aus 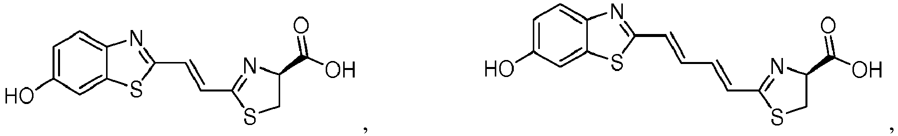 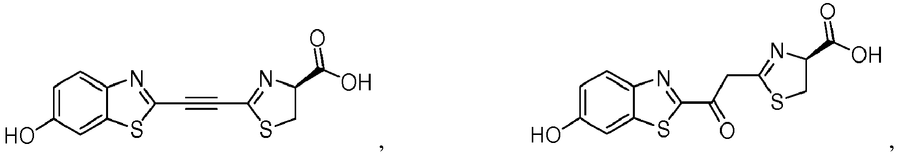 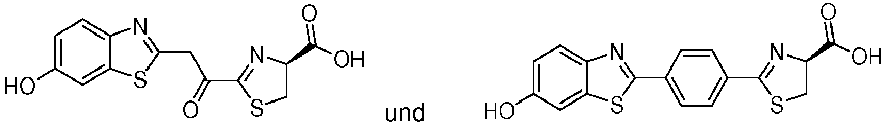 und C₁₋₆-Alkylester davon.

7. Verbindung gemäß einem vorhergehenden Anspruch zur Verwendung bei einer *In-vivo*-Bildgebung.

8. Zusammensetzung, die eine Verbindung gemäß einem der Ansprüche 1 bis 6 und einen oder mehrere zusätzliche Bestandteile umfasst.

9. Verwendung der Verbindung gemäß einem der Ansprüche 1 bis 6 als ein Biolumineszenzmarker für eine *In-Vitro*-Messung der Luciferase-Expression.

10. Verfahren einer *In-vivo*-Bildgebung, umfassend
Verabreichen einer Verbindung gemäß einem der Ansprüche 1 bis 6 und eines Luciferase-Enzyms oder eines Polynukleotids, das ein Luciferase-Enzym kodiert, an ein Subjekt, und
Messen der Emission, die aus der Wirkung des Luciferase-Enzyms auf die Verbindung resultiert.

11. Verfahren gemäß Anspruch 10, weiter umfassend Erzeugen eines Bildes, das auf den erhaltenen Messungen basiert.

12. Kit, das eine Verbindung gemäß einem der Ansprüche 1 bis 6 und ein Luciferase-Enzym oder ein Polynukleotid, das ein Luciferase-Enzym kodiert, umfasst.

## Revendications

1. Composé de formule (II) : 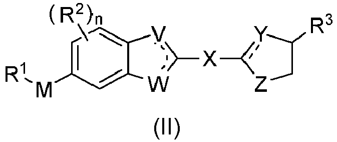 dans laquelle
R¹ est un hydrogène ou un groupe alkyle éventuellement substitué ;
chaque R², quand il est présent, est un groupe indépendamment choisi parmi hydroxyle éventuellement substitué, amino éventuellement substitué, thiol éventuellement substitué, alkyle éventuellement substitué, et aryle éventuellement substitué ;
R³ est un groupe carboxyle ou un groupe (alkyle en C₁ à C₆)carbonyloxy éventuellement substitué ;
M est un groupe choisi parmi O, S et NR⁴, où R⁴ est un groupe choisi parmi un hydrogène, alkyle éventuellement substitué, alcényle éventuellement substitué, aryle éventuellement substitué, hétéroaryle éventuellement substitué, et hétérocyclyle éventuellement substitué ;
X est un groupe choisi parmi alcénylène éventuellement substitué, alcynylène éventuellement substitué, arylène éventuellement substitué, et leurs combinaisons, ou alkylène-carbonyle éventuellement substitué ;
n vaut 0, 1, 2 ou 3 ;
chacun de V et Y est indépendamment choisi parmi CR⁵ et N ;
chacun de W et Z est indépendamment choisi parmi NR⁷, S et O ;
R⁵ est un groupe choisi parmi un hydrogène, hydroxyle éventuellement substitué, alkyle éventuellement substitué, et amino éventuellement substitué ; et
R⁷ est un groupe choisi parmi un hydrogène, alkyle éventuellement substitué, et aryle éventuellement substitué ;
où chacun des groupes éventuellement substitués listés ci-dessus est éventuellement substitué par :
(1) un groupe choisi parmi -J-aryle, -J-hétéroaryle, -J-hétérocyclyle et -J-cycloalkyle en C₃ à C₆, où J représente une liaison ou un alkylène en C₁ à C₃, et ledit aryle est choisi parmi phényle, ledit hétéroaryle est choisi parmi triazolyle, thiazolyle, thiényle, pyrazolyle, pyrimidyle, pyridazinyle, pyrazinyle, et pyridyle, ledit hétérocyclyle est choisi parmi pyrrolidinyle, azétidinyle, pyrazolidinyle, oxazolidinyle, pipéridinyle, pipérazinyle, morpholinyle, et thiazolidinyle, et ledit cycloalkyle en C₃ à C₆ est choisi parmi cyclopropyle, cyclopentyle et cyclohexyle ; ou
(2) un à trois substituants choisis parmi
(a) alkyle en C₁ à C₆ (de préférence méthyle, éthyle ou isopropyle),
(b) alcényle en C₁ à C₃ (de préférence propényle)
(c) les halogènes (de préférence Cl ou Br),
(e) halogénoalkyle en C₁ à C₆ (de préférence trifluorométhyle),
(d) cyano,
(e) amino, éventuellement mono- ou di-substitué par alkyle en C₁ à C₃, tert-butoxycarbonyle ou benzyle,
(f) alcoxy en C₁ à C₃ (de préférence méthoxy),
(g) (alkyle en C₁ à C₆)carbonyle (de préférence acétyle),
(h) (alcoxy en C₁ à C₃)carbonyle,
(i) (alkyle en C₁ à C₃)carbonyloxy, y compris carboxyle, et
(j) (alkyle en C₁ à C₃)carbamoyle, y compris carbamoyle.

2. Composé selon la revendication 1, dans lequel n vaut 0 ou 1.

3. Composé selon la revendication 1 ou 2, de formule (III) : 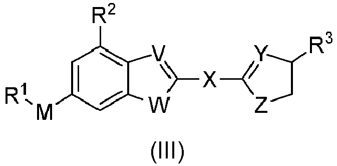 dans laquelle
chacun de V et Y est indépendamment choisi parmi CR⁵ et N ; et
chacun de W et Z est indépendamment choisi parmi NR⁷, S et O.

4. Composé selon l'une quelconque des revendications précédentes, dans lequel chacun de V et Y est N, et/ou dans lequel chacun de W et Z est S.

5. Composé selon l'une quelconque des revendications précédentes, dans lequel R¹ est l'hydrogène, et/ou
dans lequel R² est un groupe choisi parmi hydroxyle éventuellement substitué, amino éventuellement substitué, et alkyle éventuellement substitué, et/ou dans lequel X est un groupe choisi parmi alcénylène éventuellement substitué, alcynylène éventuellement substitué, et alkylène-carbonyle éventuellement substitué, de préférence dans lequel X est un groupe alkylène éventuellement substitué ; et/ou dans lequel M est O;
où les substituants éventuels de chacun des groupes éventuellement substitués sont tels que définis dans la revendication 1.

6. Composé selon l'une quelconque des revendications précédentes, choisi parmi 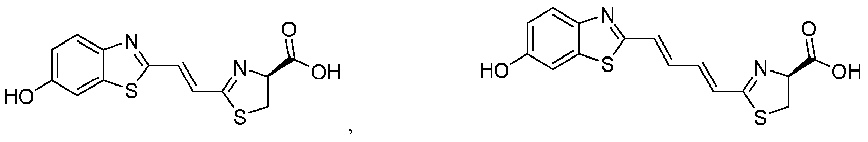 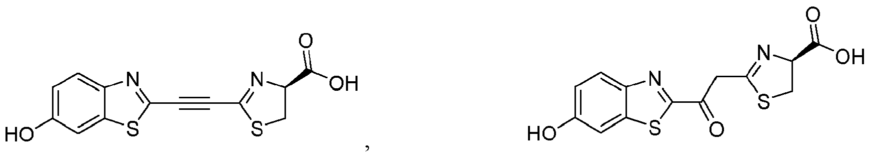 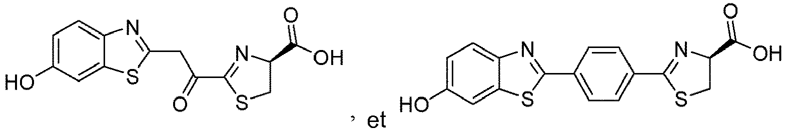 ainsi que leurs esters alkyliques en C₁ à C₆.

7. Composé selon l'une quelconque des revendications précédentes, pour une utilisation dans une imagerie in vivo.

8. Composition comprenant un composé selon l'une quelconque des revendications 1 à 6 et un ou plusieurs ingrédients additionnels.

9. Utilisation du composé selon l'une quelconque des revendications 1 à 6 en tant que marqueur bioluminescent pour la mesure in vitro de l'expression de luciférase.

10. Procédé d'imagerie in vitro comprenant
l'administration à un sujet d'un composé selon l'une quelconque des revendications 1 à 6 et d'une enzyme luciférase ou d'un polynucléotide codant une enzyme luciférase, et
la mesure de l'émission résultant de l'action de l'enzyme luciférase sur le composé.

11. Procédé selon la revendication 10, comprenant en outre la génération d'une image basée sur les mesures obtenues.

12. Trousse comprenant un composé selon l'une quelconque des revendications 1 à 6 et une enzyme luciférase ou un polynucléotide codant une enzyme luciférase.


### SEQUENCE LISTING

<110> UCL Business Plc
<120> Compounds and Their Uses
<130> P527941PCT
<150> GB1306248.4
   <151> 2013-04-05
<160> 2
<170> PatentIn version 3.5
<210> 1
   <211> 1644
   <212> DNA
   <213> Artificial Sequence
<220>
   <223> Synthetic Construct
<400> 1
   atggaagacg ccaaaaacat aaagaaaggc ccggcaccac gctatcctct agaggatgga      60
   accgctggag agcaactgca taaggctatg aagagatacg cccaggttcc tggaacaatt     120
   gcttttacag atgcacatat cgaggtgaac atcacgtacg cggaatactt cgaaatgtcc     180
   gttcggttgg cagaagctat gaaacgatat gggctgaata caaatcacag aatcgtcgta     240
   tgcagtgaaa actctcttca attctttatg ccggtgttgg gcgcgttatt tatcggagtt     300
   gcagttgcgc ccgcgaacga catttataat gaacgtgaat tgctcaacag tatgaacatt     360
   tcgcagccta ccgtagtgtt tgtttccaaa aaggggttgc aaaaaatttt gaacgtgcaa     420
   aaaaaattac caataatcca gaaaattatt atcatggatt ctaaaacgga ttaccaggga     480
   tttcagtcga tgtacacgtt cgtcacatct catctacctc ccggttttaa tgaatacgat     540
   tttaaaccag aaagctttga tcgtgacaaa acaattgcac tgataatgaa ttcctctgga     600
   tctactgggt tacctaaggg tgtggccctt ccgcatagaa ctgcctgcgt cagattctcg     660
   cacgccagag atcctatttt tggcaatcaa atcaaaccgg atactgcgat tttaagtgtt     720
   gttccattcc atcacggttt tggaatgttt actacactcg gatatttgat atgtggattt     780
   cgagtcgtct taatgtatag atttgaagaa gagctgtttt tacgatccct tcaggattac     840
   aaaattcaaa gtgcgttgct agtaccaacc ctattttcat tcttcgccaa aagcactctg     900
   attgacaaat acgatttatc taatttacac gaaattgctt ctgggggcgc acctctttcg     960
   aaagaagtcg gggaagcggt tgcaaaacgc ttccatcttc cagggatacg acaaggatat    1020
   gggctcactg agactacatc agctattctg attacacccg agggggatga taaaccgggc    1080
   gcggtcggta aagttgttcc attttttgaa gcgaaggttg tggatctgga taccgggaaa    1140
   acgctgggcg ttaatcagag aggcgaatta tgtgtcagag gacctatgat tatgtccggt    1200
   tatgtaaaca atccggaagc gaccaacgcc ttgattgaca aggatggatg gctacattct    1260
   ggagacatag cttactggga cgaagacgaa cacttcttca tagttgaccg cttgaagtct    1320
   ttaattaaat acaaaggata tcaggtggcc cccgctgaat tggaatcgat attgttacaa    1380
   caccccaaca tccgcgacgc cggcgtggca ggtcttcccg acgatgacgc cggtgaactt    1440
   cccgccgccg ttgttgtttt ggagcacgga aagacgatga cggaaaaaga gatcgtggat    1500
   tacgtcgcca gtcaagtaac aaccgcgaaa aagttgcgcg gaggagttgt gtttgtggac    1560
   gaagtaccga aaggtcttac cggaaaactc gacgcaagaa aaatcagaga gatcctcata    1620
   aaggccaaga agggcggaaa gtaa                                           1644
<210> 2
   <211> 1644
   <212> DNA
   <213> Artificial Sequence
<220>
   <223> Synthetic Construct
<400> 2
   atggaggacg ccaagaacat caagaaggga ccagccccca gataccccct ggaggacggc      60
   acagccggcg agcagctgca caaggccatg aagcggtacg cccaggtgcc aggcaccatc     120
   gccttcaccg acgcccacat cgaggtgaac atcacctacg ccgagtactt cgagatgagc     180
   gtgcggctgg ccgaggccat gaagcggtac ggcctgaaca ccaaccaccg gatcgtggtg     240
   tgcagcgaga acagcctgca gttcttcatg cccgtgctgg gagccctgtt catcggcgtg     300
   gccgtggccc cagccaacga catctacaac gagcgggagc tgctgaacag catgaacatc     360
   agccagccca ccgtggtgtt cgtgagcaag aagggcctgc agaagatcct gaatgtgcag     420
   aagaagctgc ccatcatcca gaagatcatc atcatggaca gcaagaccga ttaccagggc     480
   ttccagagca tgtacacctt cgtgaccagc cacctgcccc caggcttcaa cgagtacgac     540
   ttcaagcccg agagcttcga ccgggacaag accatcgccc tgatcatgaa cagcagcggc     600
   agcaccggcc tgcccaaggg cgtggccctg ccccaccgga ccgcctgcgt gcggttcagc     660
   cacgccagag accccatctt cggcaaccag atcatccccg acaccgccat cctgagcgtg     720
   gtgcccttcc accacggctt cggcatgttc accaccctgg gctacctgat ctgcggcttc     780
   cgggtggtgc tgatgtacag gttcgaggag gagctgttcc tgcggagcct gcaggactac     840
   aagatccaga ccgccctgct ggtgcccacc ctgttcagct tcttcgccaa gagcaccctg     900
   atcgacaagt acgacctgag caacctgcac gagatcgcct ctggcggagc cccactgagc     960
   aaggaggtgg gcgaggccgt ggccaagcgg ttccacctgc caggcatccg gcagggctac    1020
   ggcctgaccg agaccaccag cgccatcctg atcacccccg agggcgacga caagcccgga    1080
   gccgtgggca aggtggtgcc cttcttcgag gccaaggtgg tggacctgga caccggcaag    1140
   accctgggcg tgaaccagag aggcgagctg tgcgtgagag gccccatgat catgagcggc    1200
   tacgtgaaca accccgaggc caccaacgcc ctgatcgaca aggacggctg gctgcacagc    1260
   ggcgacatcg cctactggga cgaggacgag cacttcttca tcgtggaccg gctgaagagc    1320
   ctgatcaagt acaagggcta ccaggtggcc ccagccgagc tggagagcat cctgctgcag    1380
   caccccaaca tcttcgacgc cggagtggcc ggactgcccg acgacgacgc cggagagctg    1440
   ccagccgccg tggtggtgct ggagcacggc aagaccatga ccgagaagga gatcgtggac    1500
   tacgtggcca gccaggtgac caccgccaag aagctgagag gcggcgtggt gttcgtggac    1560
   gaggtgccca agggcctgac cggcaagctg gacgccagaa agatccggga gatcctgatc    1620
   aaggccaaga agggcggcaa gtaa                                           1644